(19) **Europäisches Patentamt**
**European Patent Office**
**Office européen des brevets**

(11) **EP 3 918 999 B1**

(12) **EUROPEAN PATENT SPECIFICATION**

(45) Date of publication and mention
of the grant of the patent:
**31.12.2025 Bulletin 2026/01**

(21) Application number: **21175853.7**

(22) Date of filing: **26.05.2021**

(51) International Patent Classification (IPC):
***A61B 5/367*** (2021.01)   ***A61B 5/00*** (2006.01)
***A61B 5/06*** (2006.01)   ***A61B 5/346*** (2021.01)

(52) Cooperative Patent Classification (CPC):
**A61B 5/367; A61B 5/061; A61B 5/346;**
**A61B 5/4887; A61B 5/7267; G06N 3/0442;**
**G06N 3/0464; G06N 3/09;** A61B 5/36; A61B 5/6852;
A61B 5/6869

(54) **AUTOMATIC DETECTION OF CARDIAC STRUCTURES IN CARDIAC MAPPING**

AUTOMATISCHE DETEKTION VON HERZSTRUKTUREN BEI DER HERZKARTIERUNG

DÉTECTION AUTOMATIQUE DE STRUCTURES CARDIAQUES DANS UNE CARTOGRAPHIE
CARDIAQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.05.2020 US 202063030392 P**
**24.05.2021 US 202117328216**

(43) Date of publication of application:
**08.12.2021 Bulletin 2021/49**

(73) Proprietor: **Biosense Webster (Israel) Ltd.**
**Yokneam 2066717 (IL)**

(72) Inventors:
• **Ravuna, Eliyahu**
**Yokneam (IL)**

• **Botzer, Lior**
**Yokneam (IL)**
• **Lev, Iddo**
**Tel Aviv (IL)**
• **Nakar, Elad**
**Yokneam (IL)**
• **Yarnitsky, Jonathan**
**Yokneam (IL)**

(74) Representative: **Carpmaels & Ransford LLP**
**One Southampton Row**
**London WC1B 5HA (GB)**

(56) References cited:
**WO-A1-2018/134747    US-A1- 2009 253 976**
**US-A1- 2018 296 176**

**Description**

FIELD OF INVENTION

The present disclosure is related to artificial intelligence and machine learning associated with automatically detecting the location of specific structures within the heart, and more preferably, automatically detecting the location of the His bundle in the heart.

BACKGROUND

It is well known to use ablation catheters to create tissue necrosis in cardiac tissue to correct cardiac arrhythmias (including, but not limited to, atrial fibrillation, atrial flutter, atrial tachycardia and ventricular tachycardia). Arrhythmia can create a variety of dangerous conditions including irregular heart rates, loss of synchronous atrioventricular contractions and stasis of blood flow which can lead to a variety of ailments and even death. It is believed that the primary cause of many arrhythmias is stray electrical signals within one or more heart chambers.

During a cardiac ablation, a lesion is produced in the tissue of the heart of the patient. To produce the lesion, a catheter is inserted into the heart so that it contacts the tissue, and electromagnetic radiofrequency (RF) energy is injected from a catheter electrode into the tissue, causing ablation and production of a lesion.

The His bundle, otherwise known as the bundle of His, is a part of the heart muscle that originates near the orifice of the coronary sinus (CS). The His bundle is a critical part of the electrical conduction system of the heart in that it serves a role in transmitting electrical impulses from the atrioventricular (AV) node, which located between the atria and the ventricles, to the ventricles of the heart.

The His bundle is positioned at a vulnerable location in the heart, which if ablated by mistake, could result in detrimental and unwanted effects on the electrical conduction system of the heart. During conventional ablation procedures, physicians often manually tag the His bundle to identify its location within the heart so that it can be avoided during the ablation procedure. Such manual tagging of the His bundle is tedious and time consuming. In addition, manual tagging can lead to false positive reads in which an electrocardiogram (ECG) signal appears to look like a His bundle impulse, but in reality is not.

From document US2009/253976A1 it is known to determine information about a position of an object. Current is caused to flow between each of three or more sets of current-injecting electrodes on a first catheter inserted into an organ in a patient's body, the organ having a periphery. In response to current flow caused by each set of current injecting electrodes, an electrical signal is measured at each of one or more measuring electrodes located on one or more additional catheters inserted into the organ in the patient's body. The position of each of one or more of the measuring electrodes on the additional catheters relative to the first catheter are determined based on the measured signals from the one or more measuring electrodes. In an example, the additional catheter may be placed in a stable location such as at the coronary sinus, atrial appendage or apex. A reconstruction procedure can be performed to determine physiological information at the endocardium surface from signals measured within the cardiac cavity.

A need exists for an automated and reliable system and method to automatically detect the His bundle with accuracy utilizing artificial intelligence and/or machine learning.

SUMMARY

Methods, apparatuses, systems, and models for automatically detecting the location of specific structures within the heart are described herein.

In accordance with one aspect, the subject matter disclosed herein relates to a system for automatically detecting a cardiac structure. The system preferably includes a first catheter positioned within the heart to receive electrophysiological data regarding a first cardiac structure, a second catheter located at a predetermined position within the heart; and a processor comprising a neural network. The neural network receives the electrophysiological data from the first catheter, receives distance data regarding a distance between the first catheter and the second catheter, determines whether the electrophysiological data regarding the first cardiac structure is consistent with predetermined electrophysiological data of a cardiac structure of interest, determines whether the distance between the first catheter and the second catheter is less than a predetermined threshold value, and determines whether the first cardiac structure is the cardiac structure of interest based on the electrophysiological data and the distance data.

In accordance with another aspect, the subject matter disclosed herein relates to a system for training a neural network to automatically detect a cardiac structure. The system includes a processor comprising a neural network training model that receives training data. The training data includes a location of a previously mapped cardiac structure of interest, electrophysiological data regarding a first cardiac structure received by a first catheter positioned within a heart, predetermined electrophysiological data of a cardiac structure of interest, distance data regarding the distance between the first catheter and the second catheter, and a predetermined threshold value regarding the distance between a point on the first catheter and a point on the second catheter. The neural network training model is trained to determine whether the electrophysiological data is consistent with predetermined electrophysiological data of the cardiac structure of interest, determine whether the distance data is less than the predetermined value, and determine whether the first cardiac structure is the cardiac structure of interest based on the training data.

In accordance with yet another aspect, the subject matter disclosed herein relates to a method for training a neural network model to automatically detect a cardiac structure. The method includes receiving training data by a processor comprising the neural network model. The training data includes a location of a previously mapped cardiac structure of interest, electrophysiological data regarding a first cardiac structure received by a first catheter positioned within a heart, predetermined electrophysiological data of a cardiac structure of interest, distance data regarding the distance between the first catheter and the second catheter, and a predetermined threshold value regarding the distance between a point on the first catheter and a point on the second catheter. The method further includes training the neural network model with the training data. The training includes determining whether the electrophysiological data is consistent with predetermined electrophysiological data of the cardiac structure of interest, determining whether the distance data is less than the predetermined value, and determining whether the first cardiac structure is the cardiac structure of interest based on the training data.

In accordance with yet another aspect, the cardiac structure of interest is a His bundle.

In accordance with yet another aspect, the first catheter comprises a His bundle mapping catheter.

In accordance with yet another aspect, the electrophysiological data regarding the first cardiac structure received by the first catheter comprises an electrogram, and more particularly, a His bundle electrogram.

In accordance with yet another aspect, the second catheter comprises a coronary sinus catheter, and more particular, a position sensor.

In accordance with yet another aspect, the training data further comprises electrocardiogram data generated by a surface body electrode.

In accordance with yet another aspect, the neural network is trained to determine whether the electrocardiogram data is consistent with predetermined electrophysiological data of the cardiac structure of interest.

In accordance with yet another aspect, the neural network is trained to determine that the first cardiac structure is the cardiac structure of interest when the electrophysiological data regarding the first cardiac structure is consistent with the predetermined electrophysiological data of the cardiac structure of interest and the distance data is less than the predetermined value.

In accordance with yet another aspect, the neural network is trained to determine that the first cardiac structure is not the cardiac structure of interest when the electrophysiological data regarding the first cardiac structure is inconsistent with the predetermined electrophysiological data of the cardiac structure of interest or the distance data is greater than the predetermined value.

In accordance with yet another aspect, the predetermined electrophysiological data of the cardiac structure of interest is stored in a database in communication with the neural network.

In accordance with yet another aspect, the neural network is a convolutional neural network or a long short-term memory neural network.

In accordance with yet another aspect, a neural network training model determines that the first cardiac structure is the cardiac structure of interest, the determination is compared against a database of locations of known cardiac structures to validate an accuracy of the determination. When the accuracy of the determination is above a predetermined accuracy threshold, the neural network training model is validated as a standard for cardiac mapping systems.

In accordance with yet another aspect, the subject matter disclosed herein relates to a system for training a neural network to automatically detect a cardiac structure of interest including a processor comprising a neural network training model that receives training data. The training data comprises a first input comprising first electrophysiological data regarding a first cardiac structure received by an electrode of a first catheter positioned within a heart, and a second input comprising a second data relating to the first cardiac structure. The neural network training model generates, as an output, a determination of whether the first cardiac structure is the cardiac structure of interest based on the training data.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 is a block diagram of an example system for remotely monitoring and communicating patient biometrics in accordance with the subject matter of the present application.
FIG. 2 is a system diagram of an example of a computing environment in communication with network in accordance with the subject matter of the present application.

FIG. 3 is a block diagram of an example device in which one or more features of the disclosure can be implemented in accordance with the subject matter of the present application.

FIG. 4 illustrates a graphical depiction of an artificial intelligence system incorporating the example device of FIG. 3 in accordance with the subject matter of the present application.

FIG. 5 illustrates a method performed in the artificial intelligence system of FIG. 4 in accordance with the subject matter of the present application.

FIG. 6 illustrates an example of the probabilities of a naive Bayes calculation in accordance with the subject matter of the present application.

FIG. 7 illustrates an exemplary decision tree in accordance with the subject matter of the present application.

FIG. 8 illustrates an exemplary random forest classifier in accordance with the subject matter of the present application.

FIG. 9 illustrates an exemplary logistic regression in accordance with the subject matter of the present application.

FIG. 10 illustrates an exemplary support vector machine in accordance with the subject matter of the present application.

FIG. 11 illustrated an exemplary linear regression model in accordance with the subject matter of the present application.

FIG. 12 illustrates an exemplary K-means clustering in accordance with the subject matter of the present application.

FIG. 13 illustrates an exemplary ensemble learning algorithm in accordance with the subject matter of the present application.

FIG. 14 illustrates an exemplary neural network in accordance with the subject matter of the present application.

FIG. 15 illustrates a hardware based neural network in accordance with the subject matter of the present application.

FIG. 16 shows an electrocardiogram (ECG) signal generated by contraction (depolarization) and relaxation (repolarization) of atrial and ventricular muscles of the heart in accordance with the subject matter of the present application.

FIG. 17 illustrates an exemplary cardiac ablation system in which one or more features of the disclosed subject matter can be implemented in accordance with the subject matter of the present application.

FIG. 18A illustrates a neural network, such as the neural network of FIG. 17, that receives input data to train the neural network and to automatically identify a cardiac structure of interest, such as a His bundle, with greater efficiency and reliability than manual identification by a physician during a procedure, such as an ablation procedure.

FIG. 18B is a flow diagram showing an embodiment of a module for training a neural network in accordance with the subject matter of the present application.

FIG. 19A shows a cardiac image obtained by fluoroscopy showing various catheters positioned within the heart during a cardiac ablation procedure in accordance with the subject matter of the present application.

FIG. 19B shows exemplary first and second catheters positioned within the heart in accordance with the subject matter of the present application.

FIG. 20 shows exemplary ECG and His bundle electrogram (HBE) signals that may be used according to the systems and methods of this disclosure in accordance with the subject matter of the present application.

FIG. 21 illustrates an exemplary convolutional neural network (CNN) in accordance with the subject matter of the present application.

FIG. 22 illustrates an exemplary recurrent neural network (RNN) in accordance with the subject matter of the present application.

FIG. 23 illustrates an implementation of the present system as described.

FIG. 24 illustrates an implementation of the present system as described.

FIG. 25 illustrates an implementation of the present system as described.

DETAILED DESCRIPTION

A method, system, and program is provided that provides for automatically detecting the location of specific structures within the heart, and more preferably, automatically detecting the location of the His bundle in the heart, and for training a neural network to automatically detect the location such specific structures within the heart.

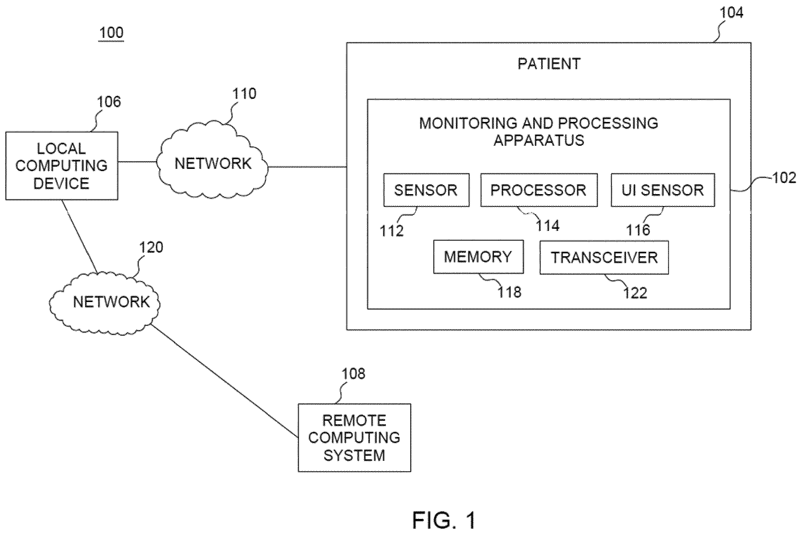

FIG. 1 is a block diagram of an example system 100 for remotely monitoring and communicating patient biometrics (i.e., patient data). In the example illustrated in FIG. 1, the system 100 includes a patient biometric monitoring and processing apparatus 102 associated with a patient 104, a local computing device 106, a remote computing system 108, a first network 110 and a second network 120.

According to an exemplary embodiment, a monitoring and processing apparatus 102 may be an apparatus that is internal to the patient's body (e.g., subcutaneously implantable). The monitoring and processing apparatus 102 may be inserted into a patient via any applicable manner including orally injecting, surgical insertion via a vein or artery, an endoscopic procedure, or a laparoscopic procedure.

According to an exemplary embodiment, a monitoring and processing apparatus 102 may be an apparatus that is external to the patient. For example, as described in more detail below, the monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an exemplary embodiment, a monitoring and processing apparatus 102 may include both components that are internal to the patient and components that are external to the patient.

A single monitoring and processing apparatus 102 is shown in FIG. 1. Example systems may, however, may include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other monitoring and processing apparatuses 102. The additional information may be, for example, diagnosis information and/or additional information obtained from an additional device such as a wearable device. Each monitoring and processing apparatus 102 may process data, including its own acquired patient biometrics as well as data received from one or more other monitoring and processing apparatuses 102.

In FIG. 1, network 110 is an example of a short-range network (e.g., local area network (LAN), or personal area network (PAN)). Information may be sent, via short-range network 110, between monitoring an processing apparatus 102 and local computing device 106 using any one of various short-range wireless communication protocols, such as Bluetooth, Wi-Fi, Zigbee, Z-Wave, near field communications (NFC), ultraband, Zigbee, or infrared (IR).

In an exemplary embodiment, network 120 may be a wired network, a wireless network or include one or more wired and wireless networks. For example, a network 120 may be a long-range network (e.g., wide area network (WAN), the internet, or a cellular network,). Information may be sent, via network 120 using any one of various long-range wireless communication protocols (e.g., TCP/IP, HTTP, 3G, 4G/LTE, or 5G/New Radio).

In an exemplary embodiment, the patient monitoring and processing apparatus 102 may include a patient biometric sensor 112, a processor 114, a user input (UI) sensor 116, a memory 118, and a transmitter-receiver (i.e., transceiver) 122. The patient monitoring and processing apparatus 102 may continually or periodically monitor, store, process and communicate, via network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

In an embodiment, patient biometric sensor 112 may include, for example, one or more sensors configured to sense a type of biometric patient biometrics. For example, patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone.

In an exemplary embodiment, as described in more detail below, patient biometric monitoring and processing apparatus 102 may be an ECG monitor for monitoring ECG signals of a heart. The patient biometric sensor 112 of the ECG monitor may include one or more electrodes for acquiring ECG signals. The ECG signals may be used for treatment of various cardiovascular diseases.

In an exemplary embodiment, transceiver 122 may include a separate transmitter and receiver. Alternatively, transceiver 122 may include a transmitter and receiver integrated into a single device.

In an exemplary embodiment, processor 114 may be configured to store patient data, such as patient biometric data in memory 118 acquired by patient biometric sensor 112, and communicate the patient data, across network 110, via a transmitter of transceiver 122. Data from one or more other monitoring and processing apparatus 102 may also be received by a receiver of transceiver 122, as described in more detail below.

According to an exemplary embodiment, the monitoring and processing apparatus 102 includes UI sensor 116 which may be, for example, a piezoelectric sensor or a capacitive sensor configured to receive a user input, such as a tapping or touching. For example UI sensor 116 may be controlled to implement a capacitive coupling, in response to tapping or touching a surface of the monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from processing apparatus 102 when a gesture is detected.

In an exemplary embodiment, the local computing device 106 of system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via network 120. Alternatively, the local computing device 106 may be a stationary or standalone device, such as a stationary base station including, for example, modem and/or router capability, a desktop or laptop computer using an executable program to communicate information between the processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some exemplary embodiments, remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

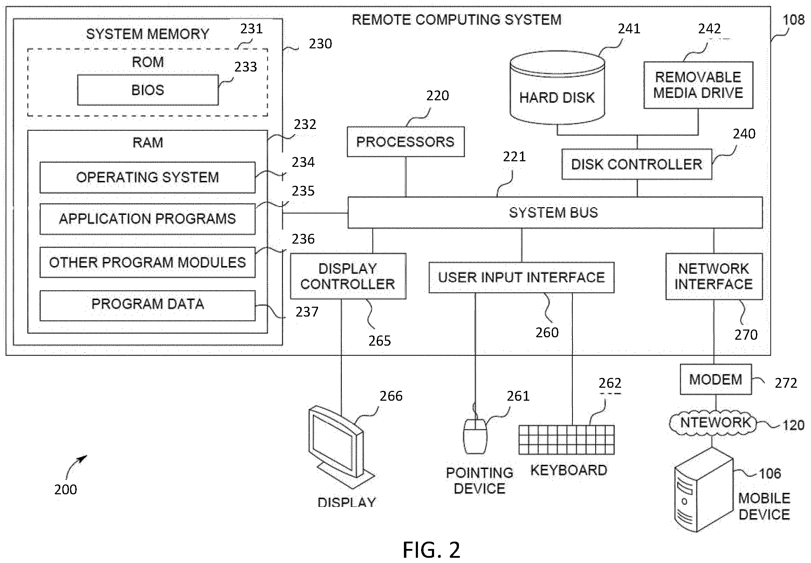

FIG. 2 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 2, computing environment 200 preferably includes remote computing system 108 (hereinafter computer system), which is one example of a computing system upon which embodiments described herein may be implemented.

The remote computing system 108 may, via processors 220, which may include one or more processors, perform various functions. For example, the functions may include analyzing monitored patient biometrics and the associated information and, according to physician-determined or algorithm driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 2, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also may include a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

In an exemplary embodiment, the computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or each of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multiprocessing arrangement to execute the one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGS. 1 and 2, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

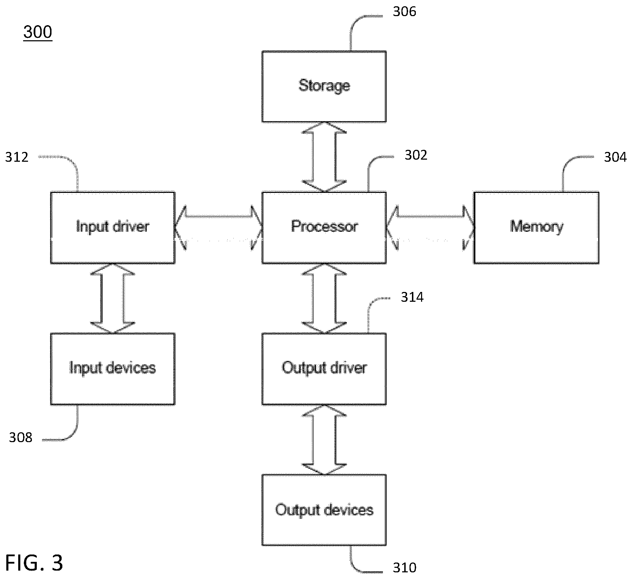

FIG. 3 is a block diagram of an example device 300 in which one or more features of the disclosure can be implemented. The device 300 may be local computing device 106, for example. The device 300 can include, for example, a computer, a gaming device, a handheld device, a set-top box, a television, a mobile phone, or a tablet computer. The device 300 includes a processor 302, a memory 304, a storage device 306, one or more input devices 308, and one or more output devices 310. The device 300 can also optionally include an input driver 312 and an output driver 314. It is understood that the device 300 can include additional components not shown in FIG. 3 including an artificial intelligence accelerator.

In various alternatives, the processor 302 includes a central processing unit (CPU), a graphics processing unit (GPU), a CPU and GPU located on the same die, or one or more processor cores, wherein each processor core can be a CPU or a GPU. In various alternatives, the memory 304 is located on the same die as the processor 302, or is located separately from the processor 302. The memory 304 includes a volatile or non-volatile memory, for example, random access memory (RAM), dynamic RAM, or a cache.

The storage device 306 includes a fixed or removable storage means, for example, a hard disk drive, a solid state drive, an optical disk, or a flash drive. The input devices 308 include, without limitation, a keyboard, a keypad, a touch screen, a touch pad, a detector, a microphone, an accelerometer, a gyroscope, a biometric scanner, or a network connection (e.g., a wireless local area network card for transmission and/or reception of wireless IEEE 802 signals). The output devices 310 include, without limitation, a display, a speaker, a printer, a haptic feedback device, one or more lights, an antenna, or a network connection (e.g., a wireless local area network card for transmission and/or reception of wireless IEEE 802 signals).

The input driver 312 communicates with the processor 302 and the input devices 308, and permits the processor 302 to receive input from the input devices 308. The output driver 314 communicates with the processor 302 and the output devices 310, and permits the processor 302 to send output to the output devices 310. It is noted that the input driver 312 and the output driver 314 are optional components, and that the device 300 will operate in the same manner if the input driver 312 and the output driver 314 are not present. The output driver 314 may include an accelerated processing device ("APD") 316 which is coupled to a display device 318. The APD accepts compute commands and graphics rendering commands from processor 302, processes those compute and graphics rendering commands, and provides pixel output to display device 318 for display. As described in further detail below, the APD 316 includes one or more parallel processing units to perform computations in accordance with a single-instruction-multiple-data ("SIMD") paradigm. Thus, although various functionality is described herein as being performed by or in conjunction with the APD 316, in various alternatives, the functionality described as being performed by the APD 316 is additionally or alternatively performed by other computing devices having similar capabilities that are not driven by a host processor (e.g., processor 302) and provides graphical output to a display device 318. For example, it is contemplated that any processing system that performs processing tasks in accordance with a SIMD paradigm may perform the functionality described herein. Alternatively, it is contemplated that computing systems that do not perform processing tasks in accordance with a SIMD paradigm performs the functionality described herein.

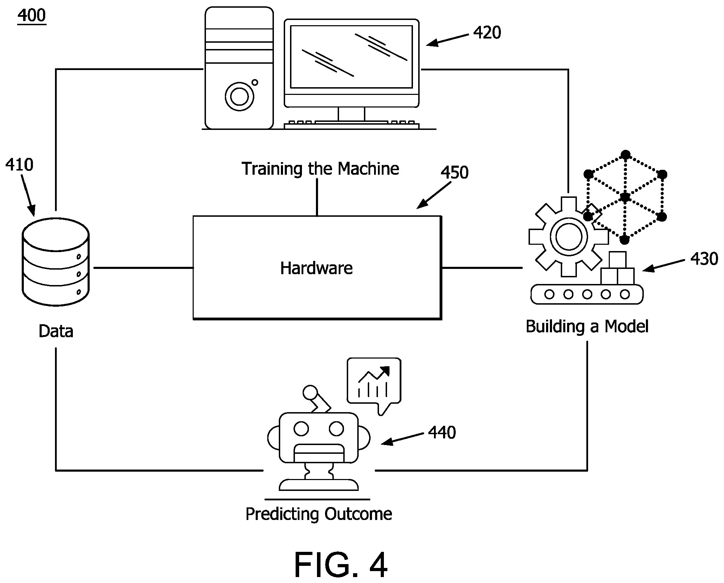

FIG. 4 illustrates a functional graphical depiction of an artificial intelligence system 400 incorporating the example device of FIG. 3. System 400 includes data 410, a machine 420, a model 430, a plurality of predicted outcomes 440 and underlying hardware 450. System 400 operates by using the data 410 to train the machine 420 while building a model 430 to enable a plurality of outcomes 440 to be predicted. The system 400 may operate with respect to hardware 450. In such a configuration, the data 410 may be related to hardware 450 and may originate with the monitoring and processing apparatus 102, for example. For example, the data 410 may be on-going data, or output data associated with hardware 450. The machine 420 may operate as the controller or data collection associated with the hardware 450, or be associated therewith. The model 430 may be configured to model the operation of hardware 450 and model the data 410 collected from hardware 450 in order to predict the outcome achieved by hardware 450. Using the predicted outcome 440, hardware 450 may be configured to provide a certain desired outcome 440 from hardware 450.

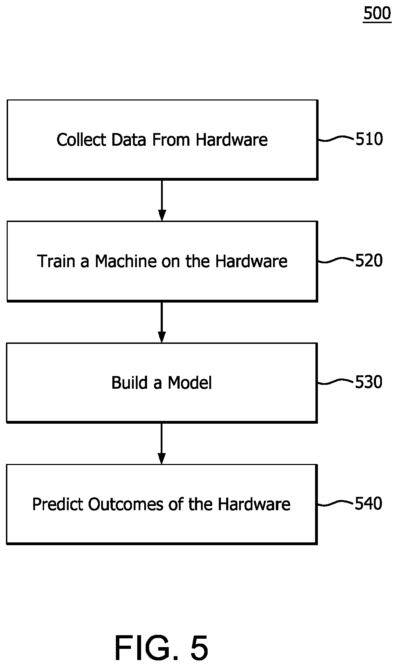

FIG. 5 illustrates a general method 500 performed in the artificial intelligence system of FIG. 4. Method 500 includes collecting data from the hardware at step 510. This data may include currently collected, historical or other data from the hardware, or various combinations thereof. For example, this data may include measurements during a surgical procedure and may be associated with the outcome of the procedure. For example, the temperature of a heart may be collected and correlated with the outcome of a heart procedure.

At step 520, method 500 includes training a machine on the hardware. The training may include an analysis and correlation of the data collected in step 510. For example in the case of the heart, the data of temperature and outcome may be trained to determine if a correlation or link exists between the temperature of the heart during the procedure and the outcome.

At step 530, method 500 includes building a model on the data associated with the hardware. Building a model may include physical hardware or software modeling, algorithmic modeling and the like, as will be described below. This modeling may seek to represent the data that has been collected and trained.

At step 540, method 500 includes predicting the outcomes of the model associated with the hardware. This prediction of the outcome may be based on the trained model. For example, in the case of the heart, if the temperature during the procedure between 97.7 - 100.2 produces a positive result from the procedure, the outcome can be predicted in a given procedure based on the temperature of the heart during the procedure. While this model is rudimentary, it is provided for exemplary purposes and to increase understanding of the present disclosure.

The present system and method operate to train the machine, build the model and predict outcomes using algorithms. These algorithms may be used to solve the trained model and predict outcomes associated with the hardware. These algorithms may be divided generally into classification, regression and clustering algorithms.

For example, a classification algorithm is used in the situation where the dependent variable, which is the variable being predicted, is divided into classes and predicting a class, the dependent variable, for a given input. Thus, a classification algorithm is used to predict an outcome, from a set number of fixed, predefined outcomes. A classification algorithm may include naive Bayes algorithms, decision trees, random forest classifiers, logistic regressions, support vector machines and k nearest neighbors.

Generally, a naive Bayes algorithm follows the Bayes theorem, and follows a probabilistic approach. As would be understood, other probabilistic-based algorithms may also be used, and generally operate using similar probabilistic principles to those described below for the exemplary naive Bayes algorithm.

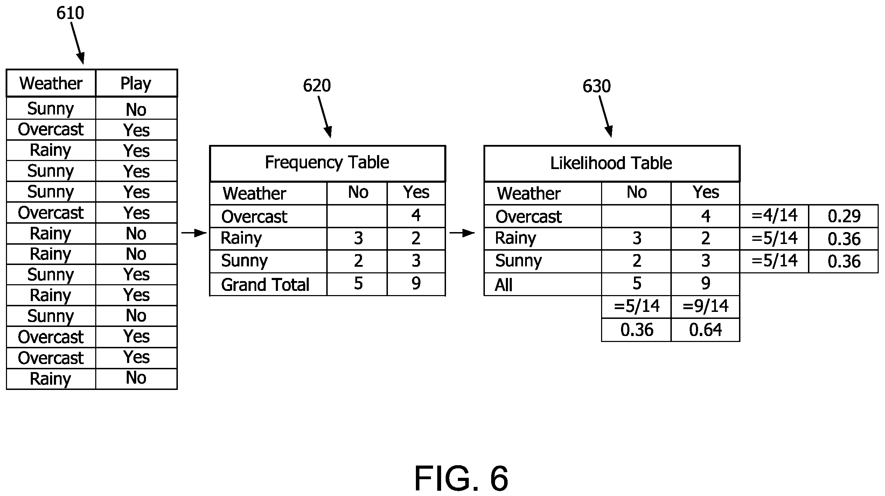

FIG. 6 illustrates an example of the probabilities of a naive Bayes calculation. The probability approach of Bayes theorem essentially means, that instead of jumping straight into the data, the algorithm has a set of prior probabilities for each of the classes for the target. After the data is entered, the naive Bayes algorithm may update the prior probabilities to form a posterior probability. This is given by the formula:

$$posterior = \frac{prior\ x\ likelihood}{evidence}$$

This naive Bayes algorithm, and Bayes algorithms generally, may be useful when needing to predict whether an input belongs to a given list of n classes or not. The probabilistic approach may be used because the probabilities for all the n classes will be quite low.

For example, as illustrated in FIG. 6, a person playing golf, which depends on factors including, without limitation, the weather outside shown in a first data set 610. The first data set 610 illustrates the weather in a first column and an outcome of playing associated with that weather in a second column. In the frequency table 620 the frequencies with which certain events occur are generated. In frequency table 620, the frequency of a person playing or not playing golf in each of the weather conditions is determined. From there, a likelihood table is compiled to generate initial probabilities. For example, the probability of the weather being overcast is 0.29 while the general probability of playing is 0.64.

The posterior probabilities may be generated from the likelihood table 630. These posterior probabilities may be configured to answer questions about weather conditions and whether golf is played in those weather conditions. For example, the probability of it being sunny outside and golf being played may be set forth by the Bayesian formula:

$$P(Yes\ |\ Sunny) = P(\ Sunny\ |\ Yes) * P(Yes)\ /\ P\ (Sunny)$$

According to likelihood table 630:

$$P\ (Sunny\ |\ Yes) = 3/9 = 0.33,$$

$$P(Sunny) = 5/14 = 0.36,$$

$$P(Yes) = 9/14 = 0.64.$$

Therefore the P(Yes | Sunny) = .33*.64/.36 or approximately 0.60 (60%).

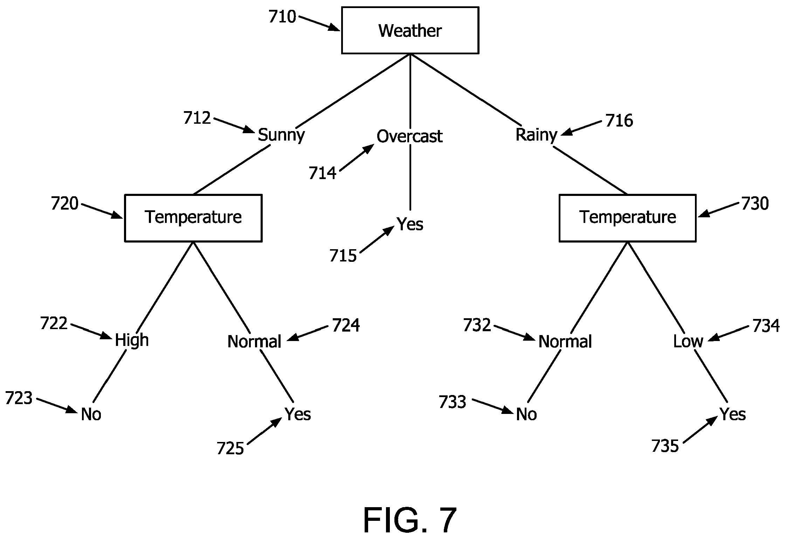

Generally, a decision tree is a flowchart-like tree structure where each external node denotes a test on an attribute and each branch represents the outcome of that test. The leaf nodes contain the actual predicted labels. The decision tree begins from the root of the tree with attribute values being compared until a leaf node is reached. A decision tree can be used as a classifier when handling high dimensional data and when little time has been spent behind data preparation. Decision trees may take the form of a simple decision tree, a linear decision tree, an algebraic decision tree, a deterministic decision tree, a randomized decision tree, a nondeterministic decision tree, and a quantum decision tree. An exemplary decision tree is provided below in FIG. 7.

FIG. 7 illustrates a decision tree, along the same structure as the Bayes example above, in deciding whether to play golf. In the decision tree, the first node 710 examines the weather providing sunny 712, overcast 714, and rain 716 as the choices to progress down the decision tree. If the weather is sunny, the leg of the tree is followed to a second node 720 examining the temperature. The temperature at node 720 may be high 722 or normal 724, in this example. If the temperature at node 720 is high 722, then the predicted outcome of "No" 723 golf occurs. If the temperature at node 720 is normal 724, then the predicted outcome of "Yes" 725 golf occurs.

Further, from the first node 710, an outcome overcast 714, "Yes" 715 golf occurs.

From the first node weather 710, an outcome of rain 716 results in the third node 730 (again) examining temperature. If the temperature at third node 730 is normal 732, then "Yes" 733 golf is played. If the temperature at third node 730 is low 734, then "No" 735 golf is played.

From this decision tree, a golfer plays golf if the weather is overcast 715, in normal temperature sunny weather 725, and in normal temperature rainy weather 733, while the golfer does not play if there is sunny high temperatures 723 or low rainy temperatures 735.

A random forest classifier is a committee of decision trees, where each decision tree has been fed a subset of the attributes of data and predicts on the basis of that subset. The mode of the actual predicted values of the decision trees are considered to provide an ultimate random forest answer. The random forest classifier, generally, alleviates overfitting, which is present in a standalone decision tree, leading to a much more robust and accurate classifier.

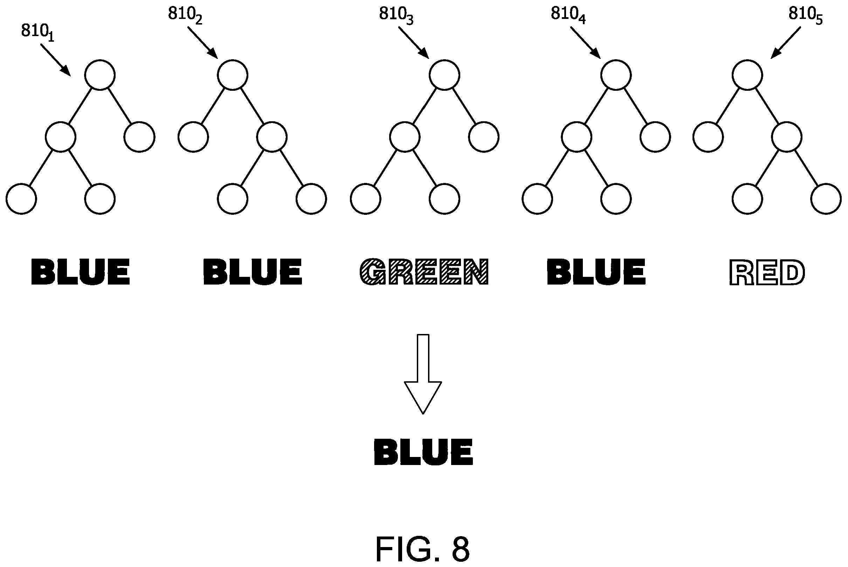

FIG. 8 illustrates an exemplary random forest classifier for classifying the color of a garment. As illustrated in FIG. 8, the random forest classifier includes five decision trees $810_1$, $810_2$, $810_3$, $810_4$, and $810_5$ (collectively or generally referred to as decision trees 810). Each of the trees is designed to classify the color of the garment. A discussion of each of the trees and decisions made is not provided, as each individual tree generally operates as the decision tree of FIG. 7. In

the illustration, three ($810_1$, $810_2$, $810_4$) of the five trees determines that the garment is blue, while one determines the garment is green ($810_3$) and the remaining tree determines the garment is red ($810_5$). The random forest takes these actual predicted values of the five trees and calculates the mode of the actual predicted values to provide random forest answer that the garment is blue.

Logistic regression is another algorithm for binary classification tasks. Logistic regression is based on the logistic function, also called the sigmoid function. This S-shaped curve can take any real-valued number and map it between 0 and 1 asymptotically approaching those limits. The logistic model may be used to model the probability of a certain class or event existing such as pass/fail, win/lose, alive/dead or healthy/sick. This can be extended to model several classes of events such as determining whether an image contains a cat, dog, lion, etc. Each object being detected in the image would be assigned a probability between 0 and 1 with the sum of the probabilities adding to one.

In the logistic model, the log-odds (the logarithm of the odds) for the value labeled "1" is a linear combination of one or more independent variables ("predictors"); the independent variables can each be a binary variable (two classes, coded by an indicator variable) or a continuous variable (any real value). The corresponding probability of the value labeled "1" can vary between 0 (certainly the value "0") and 1 (certainly the value "1"), hence the labeling; the function that converts log-odds to probability is the logistic function, hence the name. The unit of measurement for the log-odds scale is called a logit, from logistic unit, hence the alternative names. Analogous models with a different sigmoid function instead of the logistic function can also be used, such as the probit model; the defining characteristic of the logistic model is that increasing one of the independent variables multiplicatively scales the odds of the given outcome at a constant rate, with each independent variable having its own parameter; for a binary dependent variable this generalizes the odds ratio.

In a binary logistic regression model, the dependent variable has two levels (categorical). Outputs with more than two values are modeled by multinomial logistic regression and, if the multiple categories are ordered, by ordinal logistic regression (for example the proportional odds ordinal logistic model). The logistic regression model itself simply models probability of output in terms of input and does not perform statistical classification (it is not a classifier), though it can be used to make a classifier, for instance by choosing a cutoff value and classifying inputs with probability greater than the cutoff as one class, below the cutoff as the other; this is a common way to make a binary classifier.

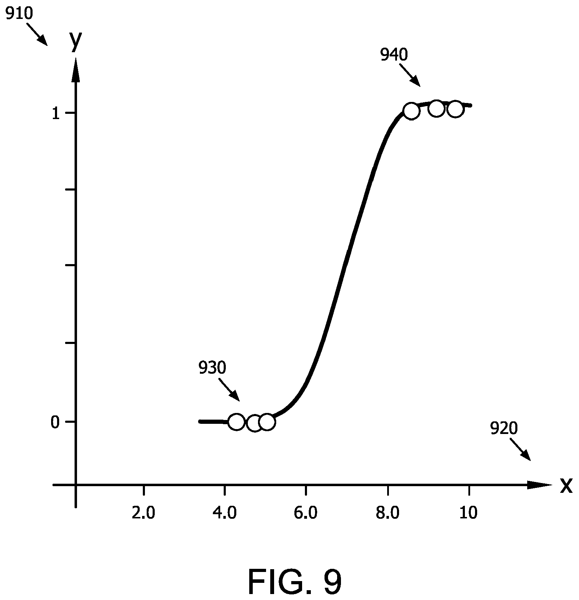

FIG. 9 illustrates an exemplary logistic regression. This exemplary logistic regression enables the prediction of an outcome based on a set of variables. For example, based on a person's grade point average, and outcome of being accepted to a school may be predicted. The past history of grade point averages and the relationship with acceptance enables the prediction to occur. The logistic regression of FIG. 9 enables the analysis of the grade point average variable 920 to predict the outcome 910 defined by 0 to 1. At the low end 930 of the S-shaped curve, the grade point average 920 predicts an outcome 910 of not being accepted. While at the high end 940 of the S-shaped curve, the grade point average 920 predicts an outcome 910 of being accepted. Logistic regression may be used to predict house values, customer lifetime value in the insurance sector, etc.

A support vector machine (SVM) may be used to sort the data with the margins between two classes as far apart as possible. This is called maximum margin separation. The SVM may account for the support vectors while plotting the hyperplane, unlike linear regression which uses the entire dataset for that purpose.

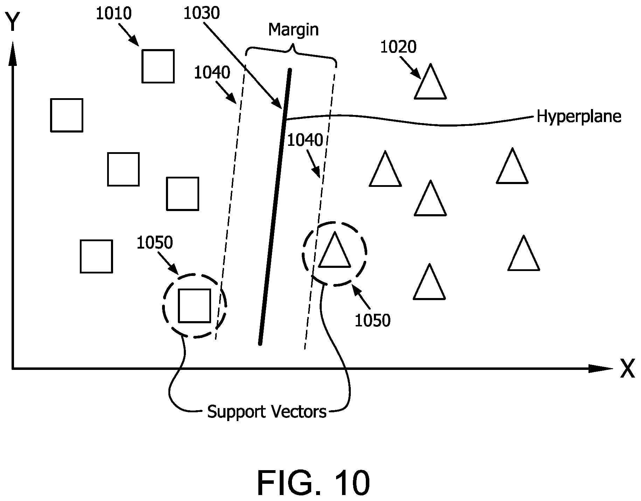

FIG. 10 illustrates an exemplary support vector machine. In the exemplary SVM 1000, data may be classified into two different classes represented as squares 1010 and triangles 1020. SVM 1000 operates by drawing a random hyperplane 1030. This hyperplane 1030 is monitored by comparing the distance (illustrated with lines 1040) between the hyperplane 1030 and the closest data points 1050 from each class. The closest data points 1050 to the hyperplane 1030 are known as support vectors. The hyperplane 1030 is drawn based on these support vectors 1050 and an optimum hyperplane has a maximum distance from each of the support vectors 1050. The distance between the hyperplane 1030 and the support vectors 1050 is known as the margin.

SVM 1000 may be used to classify data by using a hyperplane 1030, such that the distance between the hyperplane 1030 and the support vectors 1050 is maximum. Such a SVM 1000 may be used to predict heart disease, for example.

K Nearest Neighbors (KNN) refers to a set of algorithms that generally do not make assumptions on the underlying data distribution, and perform a reasonably short training phase. Generally, KNN uses many data points separated into several classes to predict the classification of a new sample point. Operationally, KNN specifies an integer N with a new sample. The N entries in the model of the system closest to the new sample are selected. The most common classification of these entries is determined and that classification is assigned to the new sample. KNN generally requires the storage space to increase as the training set increases. This also means that the estimation time increases in proportion to the number of training points.

In regression algorithms, the output is a continuous quantity so regression algorithms may be used in cases where the target variable is a continuous variable. Linear regression is a general example of regression algorithms. Linear regression may be used to gauge genuine qualities (cost of houses, number of calls, all out deals and so forth) in view of the consistent variable(s). A connection between the variables and the outcome is created by fitting the best line (hence linear regression). This best fit line is known as regression line and spoken to by a direct condition $Y = a * X + b$. Linear regression is best used in approaches involving a low number of dimensions

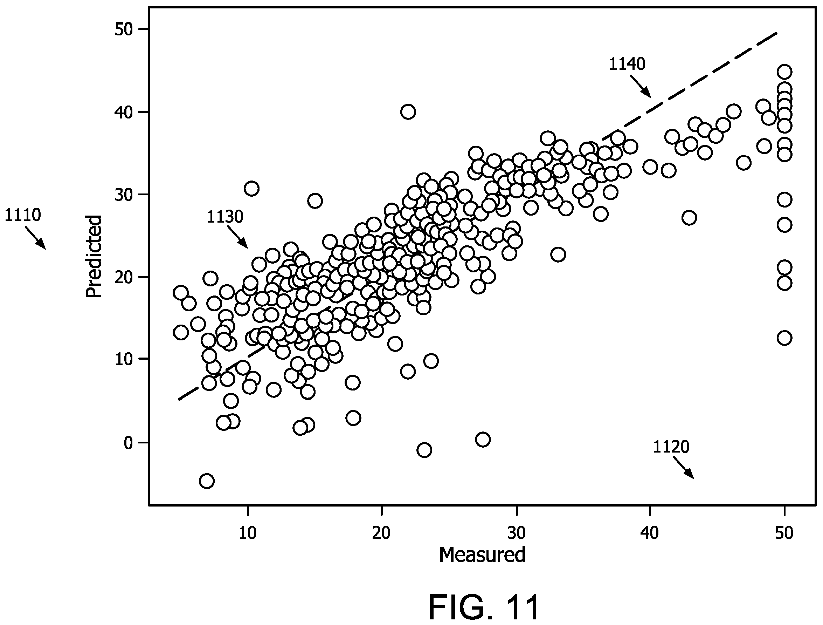

FIG. 11 illustrates an exemplary linear regression model. In this model, a predicted variable 1110 is modeled against a measured variable 1120. A cluster of instances of the predicted variable 1110 and measured variable 1120 are plotted as data points 1130. Data points 1130 are then fit with the best fit line 1140. Then the best fit line 1140 is used in subsequent predictions, given a measured variable 1120, the line 1140 is used to predict the predicted variable 1110 for that instance. Linear regression may be used to model and predict outcomes in a surgical procedure, performance of a financial portfolio, salary forecasting, real estate and in traffic in arriving at estimated time of arrival.

Clustering algorithms may also be used to model and train on a data set. In clustering, the input is assigned into two or more clusters based on feature similarity. Clustering algorithms generally learn the patterns and useful insights from data without any guidance. For example, clustering viewers into similar groups based on their interests, age, geography, etc. may be performed using unsupervised learning algorithms such as K-means clustering.

K-means clustering generally is regarded as a simple unsupervised learning approach. In K-means clustering similar data points may be gathered together and bound in the form of a cluster. One method for binding the data points together is by calculating the centroid of the group of data points. In determining effective clusters in K-means clustering the distance between each point from the centroid of the cluster is evaluated. Depending on the distance between the data point and the centroid, the data is assigned to the closest cluster. The goal of clustering is to determine the intrinsic grouping in a set of unlabeled data. The 'K' in K-means stands for the number of clusters formed. The number of clusters (basically the number of classes in which new instances of data may be classified) may be determined by the user. This determination may be performed using feedback and viewing the size of the clusters during training, for example.

K-means is used in cases where the data set has points which are distinct and well separated, otherwise, if the clusters are not separated the modeling may render the clusters inaccurate. Additionally, K-means may be avoided in cases where the data set contains a high number of outliers or the data set is non-linear.

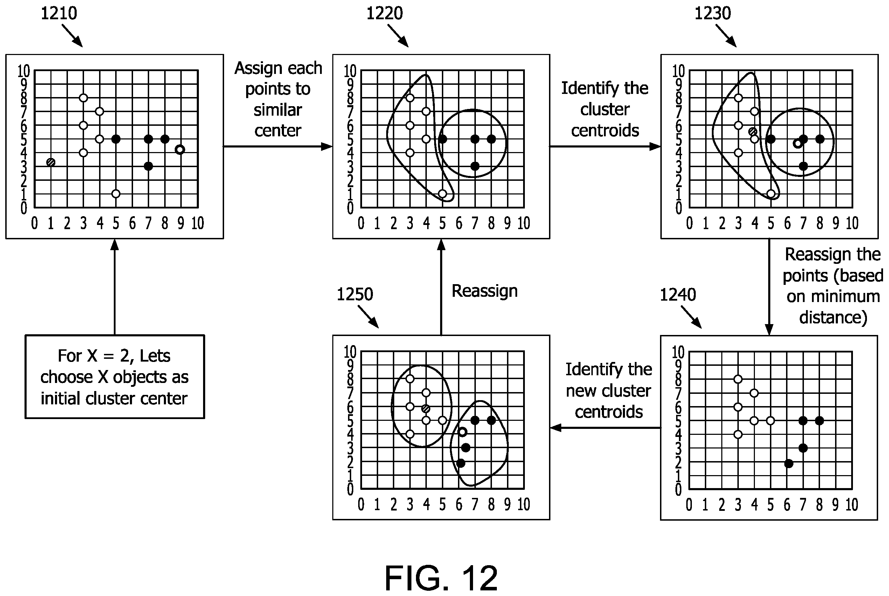

FIG. 12 illustrates K-means clustering. In K-means clustering, the data points are plotted and the K value is assigned. For example, for K=2 in FIG. 12, the data points are plotted as shown in depiction 1210. The points are then assigned to similar centers at step 1220. The cluster centroids are identified as shown in 1230. Once centroids are identified, the points are reassigned to the cluster to provide the minimum distance between the data point to the respective cluster centroid as illustrated in 1240. Then a new centroid of the cluster may be determined as illustrated in depiction 1250. As the data pints are reassigned to a cluster and new cluster centroids formed, an iteration, or series of iterations, may occur to enable the clusters to be minimized in size and the centroid of the optimal centroid determined. Then as new data points are measured, the new data points may be compared with the centroid and cluster to identify with that cluster.

Ensemble learning algorithms may be used. These algorithms use multiple learning algorithms to obtain better predictive performance than could be obtained from any of the constituent learning algorithms alone. Ensemble learning algorithms perform the task of searching through a hypothesis space to find a suitable hypothesis that will make good predictions with a particular problem. Even if the hypothesis space contains hypotheses that are very well-suited for a particular problem, it may be very difficult to find a good hypothesis. Ensemble algorithms combine multiple hypotheses to form a better hypothesis. The term ensemble is usually reserved for methods that generate multiple hypotheses using the same base learner. The broader term of multiple classifier systems also covers hybridization of hypotheses that are not induced by the same base learner.

Evaluating the prediction of an ensemble typically requires more computation than evaluating the prediction of a single model, so ensembles may be thought of as a way to compensate for poor learning algorithms by performing a lot of extra computation. Fast algorithms such as decision trees are commonly used in ensemble methods, for example, random forests, although slower algorithms can benefit from ensemble techniques as well.

An ensemble is itself a supervised learning algorithm, because it can be trained and then used to make predictions. The trained ensemble, therefore, represents a single hypothesis. This hypothesis, however, is not necessarily contained within the hypothesis space of the models from which it is built. Thus, ensembles can be shown to have more flexibility in the functions they can represent. This flexibility can, in theory, enable them to over-fit the training data more than a single model would, but in practice, some ensemble techniques (especially bagging) tend to reduce problems related to over-fitting of the training data.

Empirically, ensemble algorithms tend to yield better results when there is a significant diversity among the models. Many ensemble methods, therefore, seek to promote diversity among the models they combine. Although non-intuitive, more random algorithms (like random decision trees) can be used to produce a stronger ensemble than very deliberate algorithms (like entropy-reducing decision trees). Using a variety of strong learning algorithms, however, has been shown to be more effective than using techniques that attempt to dumb-down the models in order to promote diversity.

The number of component classifiers of an ensemble has a great impact on the accuracy of prediction. *A priori* determining of ensemble size and the volume and velocity of big data streams make this even more crucial for online ensemble classifiers. A theoretical framework suggests that there are an ideal number of component classifiers for an ensemble such that having more or less than this number of classifiers would deteriorate the accuracy. The theoretical framework shows that using the same number of independent component classifiers as class labels gives the highest accuracy.

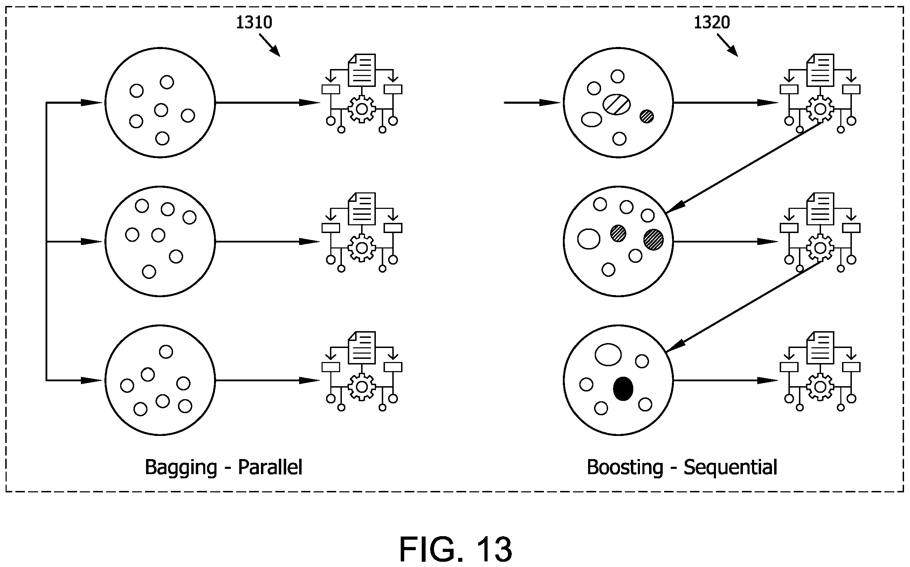

Some common types of ensembles include Bayes optimal classifier, bootstrap aggregating (bagging), boosting, Bayesian model averaging, Bayesian model combination, bucket of models and stacking. FIG. 13 illustrates an exemplary ensemble learning algorithm where bagging is being performed in parallel 1310 and boosting is being performed sequentially 1320.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms neural networks are non-linear statistical data modeling or decision making tools. They can be used to model complex relationships between inputs and outputs or to find patterns in data.

An artificial neural network involves a network of simple processing elements (artificial neurons) which can exhibit complex global behavior, determined by the connections between the processing elements and element parameters.

One classical type of artificial neural network is the recurrent Hopfield network. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which artificial neural networks are applied tend to fall within the following broad categories: function approximation, or regression analysis, including time series prediction and modeling; classification, including pattern and sequence recognition, novelty detection and sequential decision making; data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

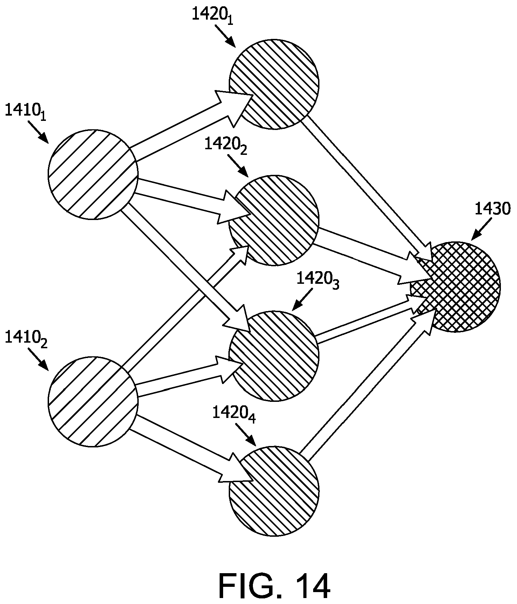

FIG. 14 illustrates an exemplary neural network. In the neural network there is an input layer represented by a plurality of inputs, such as $1410_1$ and $1410_2$. The inputs $1410_1$, $1410_2$ are provided to a hidden layer depicted as including nodes $1420_1$, $1420_2$, $1420_3$, $1420_4$. These nodes $1420_1$, $1420_2$, $1420_3$, $1420_4$ are combined to produce an output 1430 in an output layer. The neural network performs simple processing via the hidden layer of simple processing elements, nodes $1420_1$, $1420_2$, $1420_3$, $1420_4$, which can exhibit complex global behavior, determined by the connections between the processing elements and element parameters.

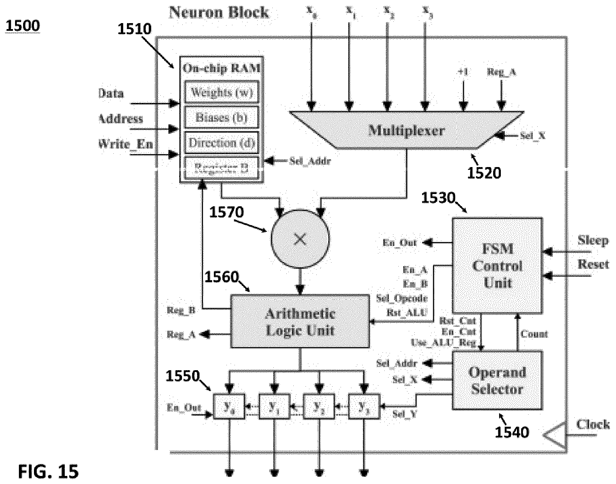

The neural network of FIG. 14 may be implemented in hardware. As depicted in FIG. 15 a hardware based neural network is depicted.

Cardiac arrhythmias, and atrial fibrillation (AF) in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, for example, in the region of the sino-atrial (SA) node, along the conduction pathways of the atrioventricular (AV) node and the His bundle, or in the cardiac muscle tissue forming the walls of the ventricular and atrial cardiac chambers.

A catheter ablation based treatment may include mapping the electrical properties of heart tissue, especially the endocardium and the heart volume, and selectively ablating cardiac tissue by application of energy. Cardiac mapping, for example, creating a map of electrical potentials (a voltage map) of the wave propagation along the heart tissue or a map of arrival times (a local time activation (LAT) map) to various tissue located points, may be used for detecting local heart tissue dysfunction. Ablations, such as those based on cardiac mapping, can cease or modify the propagation of unwanted electrical signals from one portion of the heart to another.

Cardiac ablation and other cardiac electrophysiological procedures have become increasingly complex as clinicians treat challenging conditions such as atrial fibrillation and ventricular tachycardia. The treatment of complex arrhythmias can now rely on the use of three dimensional (3D) mapping systems in order to reconstruct the anatomy of the heart chamber of interest. For example, cardiologists rely upon software such as the Complex Fractionated Atrial Electrograms (CFAE) module of the CARTO®3 3D mapping system, produced by Biosense Webster, Inc. (Diamond Bar, Calif.), to analyze intracardiac EGM signals and determine the ablation points for treatment of a broad range of cardiac conditions, including atypical atrial flutter and ventricular tachycardia. The 3D maps can provide multiple pieces of information regarding the electrophysiological properties of the tissue that represent the anatomical and functional substrate of these challenging arrhythmias.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. RF (radio frequency) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees C., a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs and the catheter must be removed from the body and the tip electrode cleaned.

A prerequisite for successfully performing a catheter ablation requires that the cause of the cardiac arrhythmia and surrounding areas of the heart are accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time.

Cardiac mapping may be implemented using one or more techniques. As an example of a first technique, cardiac mapping may be implemented by sensing an electrical property of heart tissue, for example, local activation time, as a function of the precise location within the heart. The corresponding data may be acquired with one or more catheters that are advanced into the heart using catheters that have electrical and location sensors in their distal tips. As an example, location and electrical activity may be initially measured on about 10 to about 20 points on the interior surface of the heart. These data points may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Catheters containing position sensors may be used to determine the trajectory of points on the cardiac surface.

These trajectories may be used to infer motion characteristics such as the contractility of the tissue. Maps depicting such motion characteristics may be constructed when the trajectory information is sampled at a sufficient number of points in the heart.

Electrical activity at a point in the heart may be typically measured by advancing a catheter containing an electrical sensor at or near its distal tip to that point in the heart, contacting the tissue with the sensor and acquiring data at that point. Multiple-electrode catheters may be implemented using any applicable shape such as a linear catheter with multiple electrodes, a balloon catheter including electrodes dispersed on multiple spines that shape the balloon, a lasso or loop catheter with multiple electrodes, or any other applicable shape.

According to an example, a multi-electrode catheter may be advanced into a chamber of the heart. Anteroposterior (AP) and lateral fluorograms may be obtained to establish the position and orientation of each of the electrodes. Electrograms may be recorded from each of the electrodes in contact with a cardiac surface relative to a temporal reference such as the onset of the P-wave in sinus rhythm from a body surface ECG. The system, as further disclosed herein, may differentiate between those electrodes that register electrical activity and those that do not due to absence of close proximity to the endocardial wall. After initial electrograms are recorded, the catheter may be repositioned, and fluorograms and electrograms may be recorded again. An electrical map may then be constructed from iterations of the process above..

According to another example, a technique and apparatus for mapping the electrical potential distribution of a heart chamber may be implemented. An intra-cardiac multi-electrode mapping catheter assembly may be inserted into a patient's heart. The mapping catheter assembly may include a multi-electrode array with an integral reference electrode, or, preferably, a companion reference catheter. The electrodes may be deployed in the form of a substantially spherical array. The electrode array may be spatially referenced to a point on the endocardial surface by the reference electrode or by the reference catheter which is brought into contact with the endocardial surface. The preferred electrode array catheter may carry a number of individual electrode sites (e.g., at least 24). Additionally, this example technique may be implemented with knowledge of the location of each of the electrode sites on the array, as well as a knowledge of the cardiac geometry. These locations are preferably determined by a technique of impedance plethysmography.

According to other examples, body patches and/or body surface electrodes may be positioned on or proximate to a patient's body. A catheter with one or more electrodes may be positioned within the patient's body (e.g., within the patient's heart) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient (e.g., within the heart). The biometric data may be associated with the determined position of the catheter such that a rendering of the patient's body part (e.g., heart) may be displayed and may show the biometric data overlaid on a shape of the body part, as determined by the position of the catheter.

Electrical signals such as electrocardiogram (ECG) signals are often detected prior to and/or during a cardiac procedure. For example, ECG signals can be used to identify potential locations of a heart where arrhythmia causing signals originate from. Generally, an ECG is a signal that describes the electrical activity of the heart. ECG signals may also be used to map portions of a heart.

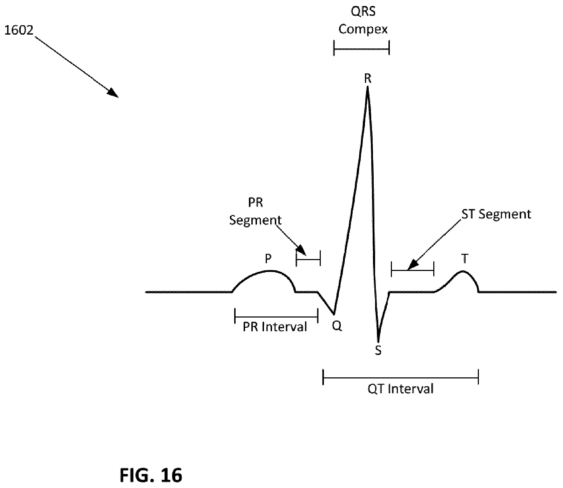

An ECG signal is generated by contraction (depolarization) and relaxation (repolarization) of atrial and ventricular muscles of the heart. As shown by signal 1602 in FIG. 16, an ECG signal contains a P wave (due to atrial depolarization), a QRS complex (due to atrial repolarization and ventricular depolarization) and a T wave (due to ventricular repolarization). In order to record an ECG signal, electrodes can placed at specific positions on the human body or can be positioned within a human body via a catheter. Artifacts (e.g., noise) are the unwanted signals that are merged with electronic signals such as ECG signals, and sometimes create obstacles for the diagnosis and/or treatment of a cardiac condition. Artifacts in electrical signals can be baseline wander, powerline interference, electromyogram (EMG) noise, power line noise, etc.

Additionally, biometric (e.g., biopotential) patient monitors may use surface electrodes to make measurements of bioelectric potentials such as ECG or electroencephalogram (EEG). The fidelity of these measurements is limited by the effectiveness of the connection of the electrode to the patient. The resistance of the electrode system to the flow of electric currents, known as the electric impedance, characterizes the effectiveness of the connection. Typically, the higher the impedance, the lower the fidelity of the measurement. Several mechanisms may contribute to lower fidelity.

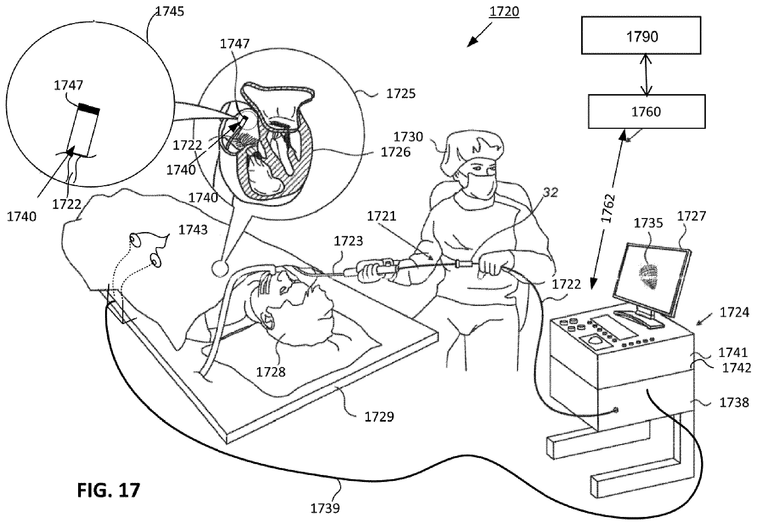

FIG. 17 is a diagram of an exemplary system 1720 in which one or more features of the disclosure subject matter can be implemented. All or parts of system 1720 may be used to collect information for a training dataset and/or all or parts of system 1720 may be used to implement a trained model. System 1720 may include components, such as a catheter 1740, that are configured to damage tissue areas of an intra-body organ. The catheter 1740 may also be further configured to obtain biometric data including electronic signals. Although catheter 1740 is shown to be a point catheter, it will be understood that a catheter of any shape that includes one or more elements (e.g., electrodes) may be used to implement the embodiments disclosed herein. System 1720 includes a probe 1721, having shafts that may be navigated by a physician 1730 into a body part, such as heart 1726, of a patient 1728 lying on a table 1729. According to embodiments, multiple probes may be provided, however, for purposes of conciseness, a single probe 1721 is described in this example, but it will be understood that probe 1721 may represent multiple probes. As shown in FIG. 17, physician 1730 may insert shaft 1722 through a sheath 1723, while manipulating the distal end of the shafts 1722 using a manipulator near the proximal end of the catheter 1740 and/or deflection from the sheath 1723. As shown in an inset 1725, catheter 1740 may be fitted at the distal end of shafts 1722. Catheter 1740 may be inserted through sheath 1723 in a collapsed state and may be then expanded within heart 1726. Cather 1740 may include at least one ablation electrode 1747 and a catheter needle, as further described herein.

According to embodiments, catheter 1740 may be configured to ablate tissue areas of a cardiac chamber of heart 1726. Inset 1745 shows catheter 1740 in an enlarged view, inside a cardiac chamber of heart 1726. As shown, catheter 1740 may include at least one ablation electrode 1747 coupled onto the body of the catheter. According to other embodiments, multiple elements may be connected via splines that form the shape of the catheter 1740. One or more other elements (not shown) may be provided and may be any elements configured to ablate or to obtain biometric data and may be electrodes, transducers, or one or more other elements.

According to embodiments disclosed herein, the ablation electrodes, such as electrode 1747, may be configured to provide energy to tissue areas of an intra-body organ such as heart 1726. The energy may be thermal energy and may cause damage to the tissue area starting from the surface of the tissue area and extending into the thickness of the tissue area.

According to embodiments disclosed herein, biometric data may include one or more of LATs, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 17, the probe 1721, and catheter 1740 may be connected to a console 1724. Console 1724 may include a processor 1741, such as a general-purpose computer, with suitable front end and interface circuits 1738 for transmitting and receiving signals to and from catheter, as well as for controlling the other components of system 1720. In some embodiments, processor 1741 may be further configured to receive biometric data, such as electrical activity, and determine if a given tissue area conducts electricity. According to an embodiment, the processor may be external to the console 1724 and may be located, for example, in the catheter, in an external device, in a mobile device, in a cloud-based device, or may be a standalone processor.

As noted above, processor 1741 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 17 may be modified to implement the embodiments disclosed herein. The disclosed embodiments may similarly be applied using other system components and settings. Additionally, system 1720 may include additional components, such as elements for sensing electrical activity, wired or wireless connectors, processing and display devices, or the like.

According to an embodiment, a display 1727 connected to a processor (e.g., processor 1741) may be located at a remote location such as a separate hospital or in separate healthcare provider networks. Additionally, the system 1720 may be part of a surgical system that is configured to obtain anatomical and electrical measurements of a patient's organ, such as a heart, and performing a cardiac ablation procedure. An example of such a surgical system is the Carto® system sold by Biosense Webster.

The system 1720 may also, and optionally, obtain biometric data such as anatomical measurements of the patient's heart using ultrasound, computed tomography (CT), magnetic resonance imaging (MRI) or other medical imaging techniques known in the art. The system 1720 may obtain electrical measurements using catheters, electrocardiograms (EKGs) or other sensors that measure electrical properties of the heart. The biometric data including anatomical and electrical measurements may then be stored in a memory 1742 of the mapping system 1720, as shown in FIG. 17. The biometric data may be transmitted to the processor 1741 from the memory 1742. Alternatively, or in addition, the biometric data may be transmitted to a server 1760, which may be local or remote, using a network 1762.

Network 1762 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 1720 and the server 1760. The network 1762 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 1762.

In some instances, the server 1760 may be implemented as a physical server. In other instances, server 1762 may be implemented as a virtual server a public cloud computing provider (e.g., Amazon Web Services (AWS)®).

According to an exemplary embodiment, the server 1760 can be implemented as, or in communication with, a processor storing a machine learning algorithm, such as a neural network 1790. In another embodiment, the neural network 1790 can be implemented in console 1724. For example, and without limitation, neural network 1790 may be implemented on one or multiple CPU processors, on one or multiple GPU processors, on one or multiple FPGA chips, or on an ASIC dedicated to perform deep learning calculations, such as the Intel® Nervana™ Neural Network Processor. According to an exemplary embodiment, neural network 1790 can be located, without limitation, in the medical procedure room, on a server or processor in a hospital or medical facility, on a remote server or processor, or in the cloud.

Control console 1724 may be connected, by a cable 1739, to body surface electrodes 1743, which may include adhesive skin patches that are affixed to the patient 1730. The processor, in conjunction with a current tracking module, may determine position coordinates of the catheter 1740 inside the body part (e.g., heart 1726) of a patient. The position coordinates may be based on impedances or electromagnetic fields measured between the body surface electrodes 1743 and the electrode 1747 or other electromagnetic components of the catheter 1740. Additionally or alternatively, location pads may be located on the surface of bed 1729 and may be separate from the bed 1729.

Processor 1741 may include real-time noise reduction circuitry typically configured as a field programmable gate array (FPGA), followed by an analog-to-digital (A/D) ECG (electrocardiograph) or EMG (electromyogram) signal conversion integrated circuit. The processor 1741 may pass the signal from an A/D ECG or EMG circuit to another processor and/or can be programmed to perform one or more functions disclosed herein.

Control console 1724 may also include an input/output (I/O) communications interface that enables the control console to transfer signals from, and/or transfer signals to electrode 1747.

During a procedure, processor 1741 may facilitate the presentation of a body part rendering 1735 to physician 1730 on a display 1727, and store data representing the body part rendering 1735 in a memory 1742. Memory 1742 may comprise any suitable volatile and/or non-volatile memory, such as random-access memory or a hard disk drive. In some embodiments, medical professional 1730 may be able to manipulate a body part rendering 1735 using one or more input devices such as a touch pad, a mouse, a keyboard, a gesture recognition apparatus, or the like. For example, an input device may be used to change the position of catheter 1740 such that rendering 1735 is updated. In alternative embodiments, display 1727 may include a touchscreen that can be configured to accept inputs from medical professional 1730, in addition to presenting a body part rendering 1735.

According to an embodiment, neural network 1790 may be provided for automatically detecting and identifying the location of specific structures within the heart, such as the His bundle. Neural network 1790 may be of the form described with respect to FIGs. 14 and 15 above.

The His bundle is a part of the heart muscle that originates near the orifice of the CS. The His bundle is a critical part of the electrical conduction system of the heart in that it serves a role in transmitting electrical impulses from the atrioventricular (AV) node, which is located between the atria and the ventricles, to the ventricles of the heart. The His bundle is positioned at a vulnerable location in the heart, which if ablated by mistake during a catheter ablation procedure, could result in detrimental and unwanted effects on the electrical conduction system of the heart. During conventional ablation procedures, physicians may manually tag the His bundle to identify its location within the heart so that the HIS bundle can be avoided during the ablation procedure. Such manual tagging of the His bundle is tedious and time consuming. Manual tagging can also lead to false positive reads in which an electrocardiogram (ECG) signal appears to look like a His bundle impulse, but the location of the impulse does not accurately correspond with the location of the His bundle. In other conventional ablation procedures, the physician may not tag the His bundle, which increases the risk for the patient during an ablation procedure.

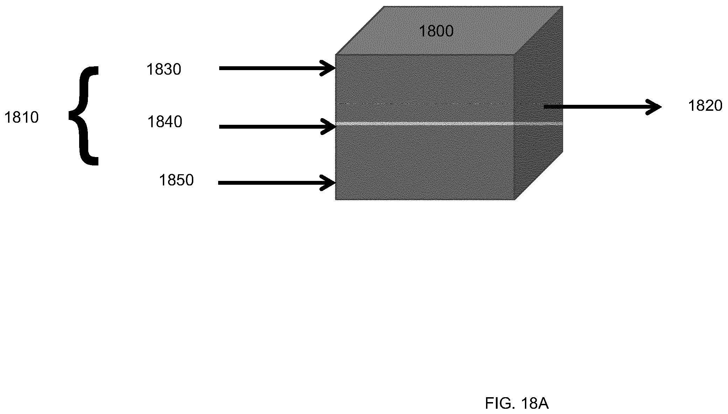

According to an exemplary embodiment in FIG. 18A, the neural network 1800, providing an illustration of the neural network 1790 of FIG. 17, receives input data 1810 to train the neural network 1800 and to automatically identify a cardiac structure of interest, such as a His bundle 1820, with greater efficiency and reliability than manual identification by a physician during a procedure, such as an ablation procedure. Non-limiting examples of the input data 1810 may include an intracardiac electrogram (EGM) or ECG signals 1830 received by an electrode, or by a bipolar electrode pair, of a catheter, such as a mapping catheter, a distance 1840 between an electrode of a first mapping catheter and a point on a second reference catheter (typically measured in millimeters), or other inputs 1850. The other inputs 1850 may include a discrete Boolean value (i.e., 0 or 1) indicating whether the distance between the electrode of the first mapping catheter and the point on the second reference catheter is less than a predetermined threshold value, a force applied by a catheter to a cardiac structure of interest as measured by a force sensor in the catheter (typically measured in grams), an index indicating the proximity between an electrode of a catheter and the cardiac structure of interest, an impedance value of the cardiac structure of interest measured by an electrode of a catheter (typically measured in Ohms), an electrocardiogram (ECG) signal 1830 received by body surface electrode(s), manual mapping data for a cardiac structure of interest, and any other electrophysiological data measured by an electrode of a catheter.

In an embodiment, one or more of the input data 1810 is fed into the neural network 1800. The input data 1810 can be stored in various locations, including, without limitation, a hospital or medical facility, at a remote server location, or in the cloud, without limitation. Training data 1810 can be transferred to neural network 1800 in real-time, at pre-determined intervals, or upon request. Once trained, the neural network 1800, can identify the His bundle 1820 in real-time during catheter ablation procedures.

In an embodiment, an output 1820 of the neural network 1800 can include, without limitation, a discrete Boolean value indicating whether the electrode of the mapping catheter is close enough to a cardiac structure of interest, such as a His bundle, and a continuous value, such as a matching index, indicating a matching between the characteristics of the cardiac of interest measured by the electrode of the mapping catheter and the characteristics of the cardiac structure of interest, such as that obtained by a manual mapping.

According to an exemplary embodiment, the neural network 1800 can comprise a convolutional neural network (CNN) or a recurrent neural network (RNN) such as a long short-term memory (LSTM) neural network. A convolutional neural network (CNN) is a deep learning algorithm preferably used in the field of computer vision and/or image recognition. A CNN assigns importance (learnable weights) to various aspects or feature in an input image in order to differentiate one from the other. An LSTM neural network is a recurrent neural network having feedback connections used for deep learning.

In an exemplary embodiment, after each training, the training model, including its output, can be executed against a standard database, such as a gold standard database, to validate its accuracy. In a non-limiting example, a gold standard database is composed of points known to be the cardiac structure in question (for example, the bundle of His), points known "not to be" this structure, relevant catheter locations, ECG signals and other relevant parameters. In an exemplary embodiment, if the accuracy of the newly trained model is below a threshold, or alternatively, if the accuracy of the newly trained model is less than the accuracy of a previous model, the model can be discarded. Similarly, if the accuracy of the newly trained model is at or above a threshold, or alternatively, if the accuracy of the newly trained model is greater than the accuracy of a previous model, the model may be published to the mapping systems in the field. In an exemplary embodiment, the publication of the new model can be performed manually, for example, by an operator downloading a file from a web address and uploading it to the mapping system 1720. Alternatively, the new model can be pushed to the mapping system 1720 in the field via the Internet.

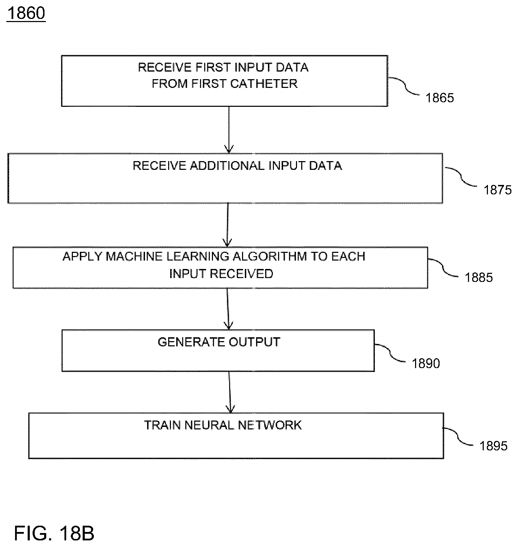

FIG. 18B is a flow diagram depicting an exemplary embodiment of a module 1860 for training a neural network 1800 and to automatically detect and identify the location of specific structures within the heart. While FIG. 18B in conjunction with FIG. 18A, depicts a module to automatically identify a His bundle in the heart, one of skill in the art will recognize that other cardiac structures or signals can be identified in accordance with module 1860.

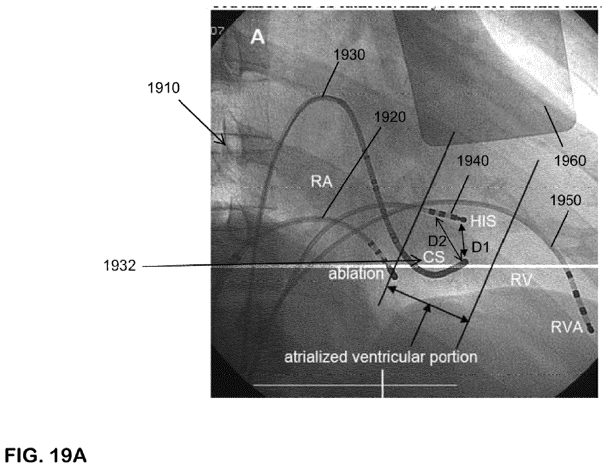

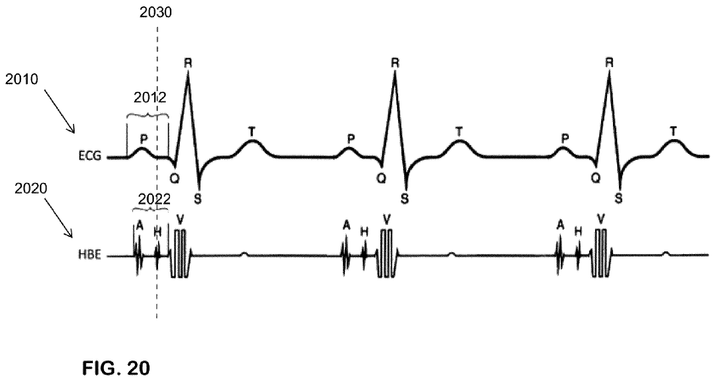

For example, during a cardiac ablation procedure, multiple catheters can be utilized to obtain various data recordings of the heart. FIG. 19A depicts multiple intracardial catheters and body surface electrodes used to obtain electrophysiological data of the heart 1910. According to an exemplary embodiment, for example, the catheters can include, without limitation, an ablation catheter 1920, a CS reference catheter 1930, and a His bundle mapping catheter 1940. Other probes may also be used as desired, such as a right ventricular apex (RVA) catheter 1950. The ablation catheter 1920 is utilized to perform an ablation procedure as discussed above. The CS reference catheter 1930 is placed inside the coronary sinus 1932 of the heart 1910. The His bundle mapping catheter 1940 contacts the His bundle and is configured to record a His bundle electrogram (HBE), as illustrated in FIG. 20, discussed herein. The body surface electrodes 1960 are positioned on the body surface body to record an ECG signal of the heart as described above and illustrated in FIGS. 16 and 20.

According to an exemplary embodiment, the electrophysiological data obtained from catheters 1920, 1930, 1940, 1950 and electrodes 1960 may be fed to processor, such as processor 1741 for analysis and output to display 1727, and are preferably transmitted to neural network 1790 as shown in FIG. 17.

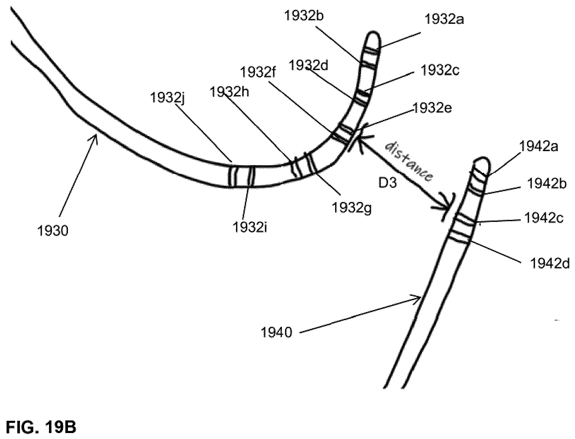

At step 1865 the neural network 1800 receives first input data from a first catheter. In an embodiment, the first input data 1810 is preferably electrophysiological data, and more preferably, an intracardiac electrogram (EGM) signal 1840 received by an electrode or bipolar electrode pair of a first catheter. In an embodiment, the first catheter is a mapping catheter that receives an EGM signal from a cardiac structure of interest. In an embodiment, the first catheter is a His bundle mapping catheter 1940, as shown in FIGS. 19A-19B, that receives a His bundle electrogram (HBE) signal. The first catheter can include multiple electrodes. FIG. 19B illustrates a His bundle mapping catheter 1940 having four electrodes 1942a, 1942b, 1942c, 1942d; however, one of skill in the art will recognize that the His bundle mapping catheter 1940 can include any number of electrodes. The His bundle mapping catheter 1940 can receive EGM signals at any of electrodes 1942a, 1942b, 1942c, 1942d.

At step 1875, the neural network 1800 receives additional input data. In an embodiment, the additional input data can be a second EGM signal received from a second electrode, such as electrode 1942b of the His bundle mapping catheter 1940. One of skill in the art will recognize that the additional input data can include multiple EGM signals received from different electrodes of the His bundle mapping catheter 1940.

To help illustrate aspects of this disclosure, FIG. 20 illustrates exemplary body surface ECG and intracardial HBE recordings 2010 and 2020, respectively, that may be used according to the methods of this disclosure. As shown in the HBE recording 2020, the A wave indicates low right atrial activation, His bundle activity is denoted H and the V deflection indicates ventricular activation. Conventionally, the time period between the onset of A wave and the subsequent onset of the V deflection is known as the AV interval 2022. When the His bundle mapping catheter 1940 contacts the His bundle, the HBE signal preferably has a pattern as shown in HBE recording 2020.

As known in the art and discussed above, the body surface electrode 1960 serves as a reference electrode and produces a body surface ECG recording 2010 of a cardiac cycle that includes a P wave, a QRS complex and a T wave as shown. The P wave represents the polarization stage of the atrial chambers, the QRS complex represents the repolarization of the ventricle, and the T wave represents depolarization of the ventricle. Correspondingly, the time period between the onset of the P wave and the onset of the QRS complex is known as the PR interval 2012. Line 2030 illustrates the point where an electrical impulse passes the His bundle in the body surface ECG recording 2010.

In an embodiment, the neural network 1800 identifies electrophysiological data, such as an HBE signal, corresponding to the location of the His bundle based on the input data. For example, the neural network 1800 identifies whether the input data includes electrophysiological data that corresponds with a His bundle.

However, even when the HBE signal has a pattern as shown in HBE recording 2020, it is possible that the His bundle mapping catheter 1940 may show a false positive reading, such as when the His bundle mapping catheter 1940 is close to, but not contacting, the His bundle. Therefore, the neural network can also rely on a distance between an electrode of the His bundle mapping catheter 1940 and the CS reference electrode 1930, as discussed herein.

In an embodiment, the additional input data can also include the distance between an electrode on the His bundle mapping catheter 1940 and an electrode on a reference catheter. For example, the reference catheter can be a CS reference catheter 1930 that is inserted into the coronary sinus of the heart as shown in FIGS. 19A-19B. For example, it is well understood that the His bundle is anatomically positioned close to the CS. According to an exemplary embodiment, the distance between an electrode on the His bundle mapping catheter 1940 and an electrode on the CS reference catheter 1930 is used as proximity data to determine the reliability of the HBE recording 2020.

In an embodiment, the CS reference catheter 1930 can include multiple electrodes. FIG. 19B illustrates a CS reference catheter 1940 having ten electrodes 1932a, 1932b, 1932c, 1932d, 1932e, 1932f, 1932g, 1932h, 1932i, 1932j; however, one of skill in the art will recognize that the CS reference catheter 1940 can include any number of electrodes. In an embodiment, the distance can be measured between each electrode 1942a-d of the His bundle mapping catheter 1940 and the closest point on the CS reference catheter 1930. For example, as shown in FIG. 19B, distance D3 is a distance between the electrode 1942c of the His bundle mapping catheter 1940 and the closest point on the CS reference catheter 1930. Alternatively, or in addition, the distance can be measured between each electrode 1942a-d of the His bundle mapping catheter 1940 and the closest electrode 1932a-j of the CS reference catheter 1930. Alternatively, or in addition, the distance can be measured between each electrode 1942a-d of the His bundle mapping catheter 1940 and a selected electrode of the CS reference catheter 1930, which could be any of one of electrodes 1932a-j.

One of skill in the art will recognize that first input data and the additional input data referenced at steps 1865 and 1885 can be any of the input data discussed herein and any other electrophysiological data measured by an electrode of a catheter.

At step 1885, the neural network 1800 applies a machine learning algorithm to each received input data to identify the location of the cardiac structure of interest, such as a His bundle. For example, the EGM received by each electrode 1942a-d of the His bundle mapping catheter 1940 and the distance between each electrode 1942a-d and the closest point to the CS reference catheter 1930 can be used to determine whether any of the electrodes 1942a-d is positioned on the His bundle, and if so, which one. For example, as shown in FIG. 19B, the EGM received by electrode 1942c of the His bundle mapping catheter 1940 and the distance D3 between electrode 1942c and the CS reference catheter 1930 can be used as inputs for the neural network 1800 to determine whether electrode 1942c of the His bundle mapping catheter 1940 is positioned on the His bundle. As is described in greater detail below, the determination of whether the electrode is positioned on the His bundle may include a review of ECG signals, distance from the CS catheter, and other inputs including force, touch status, for example.

In another example, if the HBE recording 2020 at first selected electrode of the His bundle mapping catheter 1940 has the characteristics of a His bundle, but the spatial location of the first selected electrode has a distance greater than a predetermined threshold value or range from the CS reference catheter 1930, as shown with reference to distance D2 in FIG. 19A, the neural network 1800 determines that the first selected electrode is not an accurate location of the His bundle. On the other hand, if the HBE recording 2020 has the characteristics of a His bundle, and the spatial location of a second selected electrode along the His bundle mapping catheter 1940 has a distance less than a predetermined threshold value or range from the CS reference catheter 1930, as shown with reference to distance D1 in FIG. 19A, the neural network 1800 determines that the second selected electrode is an accurate location of the His bundle.

In an embodiment, the neural network learns the predetermined threshold value based on the location of the His bundle manually marked by a physician, the distance of the manually marked His bundle to the CS reference catheter 1930, and the electrophysiological data, such as HBE recording 2020 or ECG recording 2010.

As a result, the neural network 1800 learns to automatically detect the location of specific structures within the heart, such as the His bundle based on input data such as electrophysiological received from a first catheter, such as EGM data received by an electrode of a His bundle mapping catheter 2040, and optionally additional data, such as EGM data received from other electrodes of the first catheter, electrophysiological data received from a second catheter, such as a CS reference catheter, manual mapping data, ECG data, EGM data, distance data, force data, proximity index data, impedance data, and any other electrophysiological data measured by an electrode of a catheter. This additional data may be relevant in detecting the His bundle, and although the additional data may appear unimportant, the AI algorithm described herein may find correlation and importance with ones of these input data.

At step 1890, the neural network 1800 generates an output of whether an electrode of the first catheter is positioned on, or close enough to, the cardiac structure of interest, such as a His bundle. As discussed above, the output of the neural network can include, without limitation, a discrete Boolean value indicating whether the electrode of a catheter is close enough to a cardiac structure of interest, such as a His bundle, or a continuous value, such as a matching index, indicating a matching between the characteristics of a cardiac structure of interest measured by an electrode of a catheter and the characteristics of the cardiac structure of interest, such as that obtained by a manual mapping.

In another embodiment, the neural network 1800 can be used to detect Local Abnormal Ventricular Activations (LAVA) signals within the heart. In such an embodiment, the LAVA signals and non-LAVA signals are used as training data in the model, and the neural network learns to differentiate between LAVA and non-LAVA signals to automatically detect LAVA signals in a clinical environment.

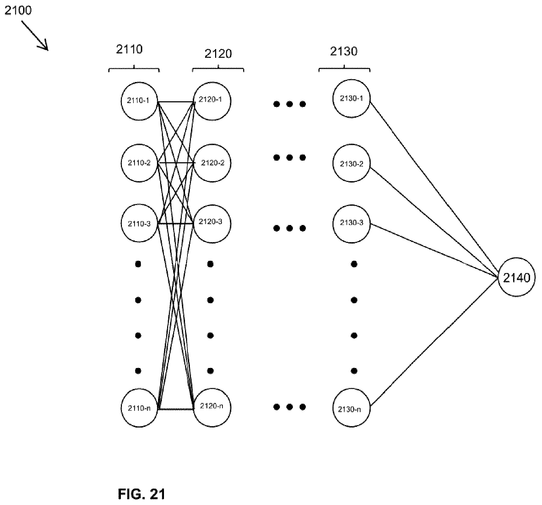

An exemplary embodiment of a convolutional neural network (CNN) 2100 for automatically identifying a cardiac structure of interest is depicted in FIG. 21. As shown in FIG. 21, the CNN 2100 preferably receives input data 2110. The input data can include a single input or a plurality of inputs 2110-1, 2110-2, 2110-3, ..., 2110-n, wherein "n" is the last of the plurality of inputs. By way of example and without limitation, a first input 2110-1 can comprise a first EGM signal received by a first electrode of a mapping catheter, a second input 2110-2 can comprise a second EGM signal received by a second electrode of the mapping catheter, a third input 2110-3 can include a distance between the first electrode of the mapping catheter and a closest electrode of a reference catheter, a last input (2110-n) can include a distance between the second electrode of the mapping catheter and a closest electrode of the reference catheter. The inputs 2110 are provided to a first hidden layer 2120 including nodes 2120-1, 2120-2, 2120-3, ... 2120-n, and optionally, to a second or more hidden layers 2130 including nodes 2130-1, 2130-2, 2130-3, ... 2130-n, which are combined to produce an output 2140, such as a Boolean value or a matching index. For example, in CNN 2100, all EGM inputs 2110 are fed to the neural network at once to calculate the output 2140. The neural network may include a series of convolution layers that feed one or more pooling layers and flattening layers to provide output in the hidden layers, for example, as will be described with more specificity below,

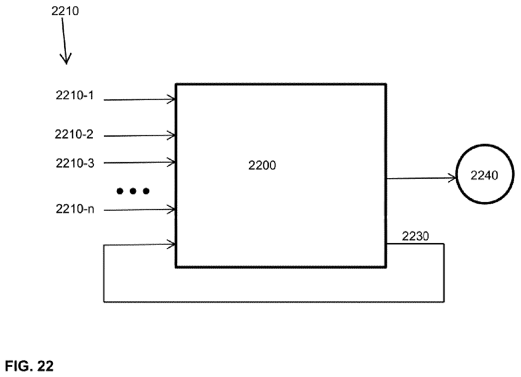

An exemplary embodiment of a recurrent neural network (RNN) 2200, such as a long short-term memory (LSTM) neural network, for automatically identifying a cardiac structure of interest is depicted in FIG. 22. As shown in FIG. 22, the RNN 2200 preferably receives input data 2210. The input data 2210 can include a single input or a plurality of inputs 2210-1, 2210-2, 2210-3, ..., 2210-n, wherein "n" is the last of the plurality of inputs. By way of example and without limitation, a first input 2210-1 can comprise a first EGM signal received by a first electrode of a mapping catheter, a second input 2210-2 can comprise distance between the first electrode of the mapping catheter and a closest electrode of a reference catheter, a third input 2210-3 can include force data received from an electrode of the mapping catheter, a last input 2210-n can include impedance data received from an electrode of the mapping catheter. The inputs 2210 are provided to the RNN 2200, and are combined to produce an output 2240, such as a Boolean value or a matching index. For example, in RNN 2200, EGM inputs 2210-1 are fed to the neural network one at a time. As more EGM samples are fed to the RNN 2200, the output 2240 becomes more accurate.

At step 1895 in FIG. 18B, the output of the neural network 1800, such as output 2140 or 2240, is used to train the neural network 1800. Specifically, the output of the neural network 1800 provides a system output, while this output is further provided to train the neural network 1800 recursively to achieve improved outputs. For example, as discussed above, after each training, the training model, including its output, can be executed against a standard database, such as a gold standard database, to validate its accuracy.. For example, the output may be a valid output, showing where the His bundle is, or is not, and the output may be used to further train the algorithm. In addition, as shown with reference to arrow 2230 in FIG. 22, if the accuracy of the newly trained model is at or above a threshold, or alternatively, if the accuracy of the newly trained model is greater than the accuracy of a previous model, the model can be used as an input for the neural network.

In an embodiment, training of the neural network 1800 can be supervised at the facility where the cardiac procedure is taking place, such as a hospital or medical facility, or at a remote location, such as a training center.

Once the neural network 1800 is trained, the neural network 1800 can be utilized in real-time to automatically detect the location of specific structures within the heart, such as the His bundle.

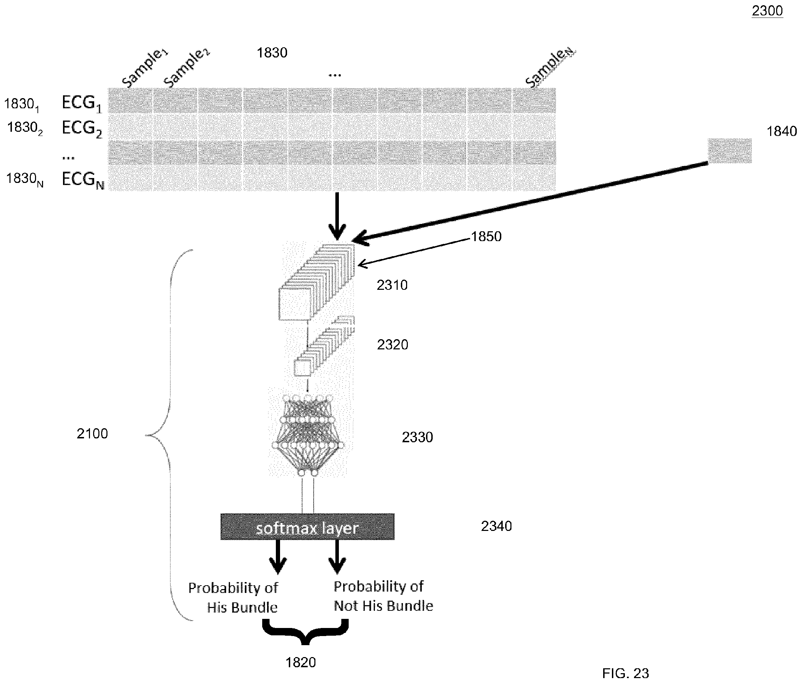

FIG. 23 illustrates an implementation 2300 of the present system as described. Implementation 2300 includes a series of inputs, including ECG inputs 1830, distance from catheter 1840 and other inputs 1850, to network 2100 to produce output 1820 including the probability of HIS bundle detection (or location)and probability of not HIS bundle detection )or location. As described hereinbefore, ECG inputs 1830 may include any number of ECG data including a first ECG $1830_1$, a second ECG $1830_2$, ..., to last ECG $1830_N$.

Network 2100 may be a CNN network, for example, as described herein. For simplicity, network 2100 may include a plurality of convolution layers 2310 interconnected with a plurality of pooling layers 2320 and further interconnected with a plurality of flattening layers 2330 that may include one or more resent and/or fully connected layers. As would be understood, a Softmax layer 2340 may be the last layer in network 2100.

Convolution layers 2310 have been described herein at least including with respect to FIG. 21. Pooling layers 2320 may be used to reduce the dimensions of the feature maps. Pooling layers 2320 may be used to reduce the number of parameters to learn and the amount of computation performed in the network 2100. The pooling layer 2310 summarizes the features present in a region of the feature map generated by convolution layer 2310. Flattening layers 2330 convert the pooled feature map to a single column that is passed to the fully connected layer and adds the fully connected layer to the neural network 2100.

Softmax layer 2340 provides a function that turns a vector of K real values into a vector of K real values that sum to 1. The input values can be positive, negative, zero, or greater than one. Softmax layer 2340 may transform the inputs to Softmax layer 2340 into values between 0 and 1 to allow interpretation as probabilities. If one of the inputs is small or negative, the Softmax layer 2340 may convert the input into a small probability, and if an input is large, then the Softmax layer 2340 turns the input into a large probability.

Softmax layer 2340 may be referred to as a softargmax function, or multi-class logistic regression. Softmax layer 2340 may be a generalization of logistic regression that can be used for multi-class classification, and its formula is very similar to the sigmoid function which is used for logistic regression. Softmax layer 2340 function can be used in a classifier only when the classes are mutually exclusive.

Softmax layer 2340 converts the scores to a normalized probability distribution, which can be displayed to a user or used as input to other systems. Softmax layer 2340 may be the final layer of the neural network 2100 to produce output 1820 including probability of HIS bundle and probability of not HIS bundle.

By feeding the distance 1840 directly as an input to the network 2100, the training may require additional time.

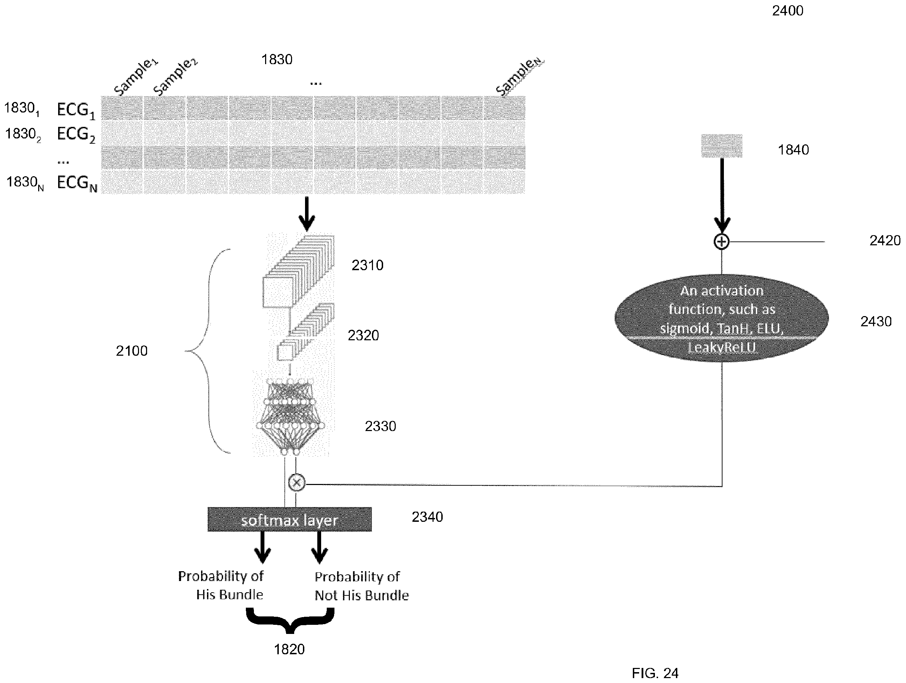

FIG. 24 illustrates an implementation 2400 of the present system as described. Implementation 2400 includes a series of inputs, including ECG inputs 1830, to network 2100 to produce output 1820 including probability of HIS bundle and probability of not HIS bundle. ECG inputs 1830 may include any number of ECG data including a first ECG $1830_1$, a second ECG $1830_2$, ..., to last ECG $1830_N$ as described above.

Network 2100 may be a CNN network, for example, as described herein. For simplicity, network 2100 may include a plurality of convolution layers 2310 interconnected with a plurality of pooling layers 2320 and further interconnected with a plurality of flattening layers 2330 that may include one or more resnet and/or fully connected layers. As would be understood, a Softmax layer 2340 may be the last layer in network 2100.

ECG inputs 1830 may be provided within network 2100 ending prior to the Softmax layer 2340.

The input of distance from catheter 1840 may be provided as an input separately from ECG inputs 1830. After multiplying by the weight and adding the bias 2420, the distance input 1840 may be provided to an activation function 2430. If d is the distance, b is the bias, w is the weight, f is the activation function, the output of the activation function is f(wd+b). As is understood in the art, activation function 2430 defines the output of that node given an input or set of inputs. Activation function 2430 may include functions such as the sigmoid, TanH, ELU and LeakyReLU, for example. The input of the softmax layer 2340 may be multiplied by the output of the activation function 2430. Softmax layer 2340 converts the scores to a normalized probability distribution, which can be displayed to a user or used as input to other systems. Softmax layer 2340 may be the final layer of the neural network 2100 to produce output 1820 including probability of HIS bundle and probability of not HIS bundle. Alternatively, instead of a softmax layer 2340 that takes two inputs and gives two outputs, a single output between $-\infty$ and $+\infty$ may be generated by the neural network 2330, and this output may be transformed to a probability between 0 and 1 by an activation function, such as the sigmoid. In this configuration, the input of the final activation function may be multiplied by the output of the activation function 2430.

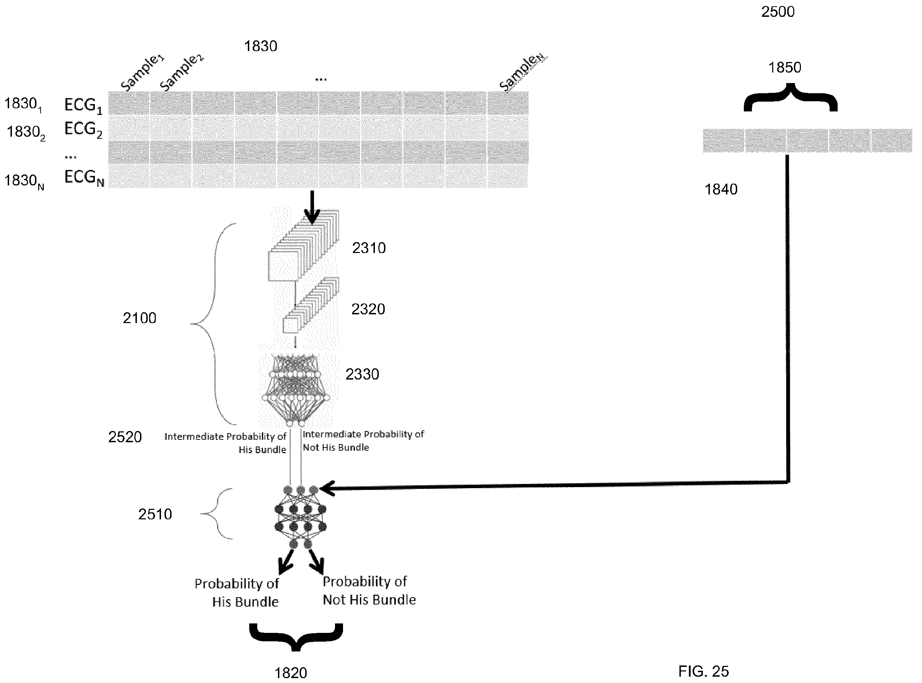

FIG. 25 illustrates an implementation 2500 of the present system as described. Implementation 2500 includes a series of inputs, including ECG inputs 1830, to network 2100 to produce output 1820 including probability of HIS bundle and probability of not HIS bundle. ECG inputs 1830 may include any number of ECG data including a first ECG $1830_1$, a second ECG $1830_2$, ..., to last ECG $1830_N$ as described above. Network 2100 may output an intermediate probability 2520 of the HIS bundle.

Network 2100 may be a CNN network, for example, as described herein. For simplicity, network 2100 may include a plurality of convolution layers 2310 interconnected with a plurality of pooling layers 2320 and further interconnected with a plurality of flattening layers 2330 that may include one or more resnet and/or fully connected layers.

The input of distance from catheter 1840 and other inputs 1850 may be provided after the convolution layers 2310, pooling layers 2320, and flattening layers 2330 of the convolutional network. As illustrated, all three layers 2310, 2320, 2330 may be skipped with distance 1840 and other inputs 1850, or just portions of layers 2310, 2320, 2330, as would be understood. The distance input 1840, and any other inputs 1850, may be provided to the ECG network 2100 output and combined using a non-convolutional neural network with one or more hidden layers 2500 to produce to produce output 1820 including probability of HIS bundle and probability of not HIS bundle. In this network architecture, since the inputs are scalar, for example distance and applied force, the inputs may be treated as "just another input" causing the training of this network to take too long, and the convergence of this network would be challenging. The configurations described in FIG. 24 and FIG. 25 resolve this problem.

While two networks 2100, 2510 are illustrated, these networks are trained monolithically as a single network. Assuming that two locations in the heart are explored with one being the desired location (the His bundle) and the other is just any point in some remote location in the heart. For the sake of the example, assume the ECG signals received from these two locations are very similar, and the only way to understand which one is the His bundle, is to look into the distance. The first neural network is not provided information on distance since the distance is not an input. If the first network is trained alone, two very similar signals would need to be fed into the neural network, once as a His bundle, once as a "Not His Bundle" causing confusion in the neural network. The neural network would not converge.

In this example, in order to provide a better understanding, the example included two very similar signals. The same problem would still exist even with different signals, because the neural network may learn some "attribute" of the signal to determine the His bundle, and this attribute can also exist in another location. Although a human would understand the difference, the neural network that inputs the ECG as the single input may be confused and its convergence would be challenging. In the best case, the training would take too long, in the worst case the network would not converge.

In training the two networks as a monolithic whole, the first network has the freedom to give a "trash" output when the distance is too large and still converge. In FIG. 24, the multiplication can reduce the effect of the "trash" when the distance is too large. In FIG. 25, the second neural network can reduce the effect of the "trash" when the distance and other parameters indicate that the probability of a His bundle is low. And, unlike a handcrafted algorithm, the network learns what a relevant distance is. The network may learn that the activation function 2430 needs to output a "close to zero" factor when the distance is large enough. The "close to zero" factor may cause the probability of the His bundle to be output as low by the second network, regardless of the output of the first network.

Generally, a cutoff does not aid in the outcome. For example, if the ECG signals where the distance was <1 cm are considered, and all other signals excluded during the training phase. Theoretically, such a configuration and cutoff provide the same "fast convergence" advantage and this would also solve the problem. The effect of the distance should be a continuous function, not a discrete function. The effect of the architecture in FIG. 24 and FIG.25 is, as-if the neural network learns to build a function to represent the effect of the distance. During the training, the network gives less and less importance to the inputs of far-away points. And this solution is scalable to any additional scalar inputs, such as the applied force, the tissue proximity index etc.

In FIG.25, the first network may output more outputs to indicate different attributes of the signal, and the second network may learn to combine the attributes with the distance. For example, when the distance is low, the second network may learn to give more importance to the attributes, such as attributes A, B and C. When the distance is high, the second network may learn to give more importance to the attributes, such as attributes D and E.

The first part of the network 2100 may or may not finish with a softmax or sigmoid layer. If it finishes with a softmax or sigmoid layer, the input to the second part of the network is between 0 and 1. Otherwise, the input to the second part of the network is between $-\infty$ and $+\infty$.

While automatic detection of the His bundle is described herein as a result of utilizing the neural network described herein, the subject matter of the present disclosure is not limited to automatic detection of the His bundle. Automatic identification of other cardiac structures and/or signals is within the scope of the disclosed subject matter. For example, the electro-cardiac cycle starts with the sinoatrial (SA) node transmitting an electrical impulse through the atria and through the atrioventricular (AV) node to the His bundle. The His bundle transmits electrical impulses from the AV node to left and right bundle branches, then to Purkinje fibers, which provide the electrical signal to the ventricles. In an embodiment, the subject matter disclosed herein can be used to automatically detect other cardiac structures in the electro-cardiac cycle including, without limitation, the SA node, the left and right bundle branches, Purkinje fibers, etc. In addition, as another example, the subject matter disclosed herein can be used to detect LAVA signals as previously discussed. In another embodiment, the subject matter disclosed herein can be used as to detect the position of a catheter and serve as an alert system for physicians to detect when a catheter unintentionally shifts from an atrial chamber to a ventricle chamber.

Although features and elements are described above in particular combinations, one of ordinary skill in the art will appreciate that each feature or element can be used alone or in any combination with the other features and elements. In addition, although process steps are described above in a particular order, the steps can be performed in other desirable orders.

The methods, processes, modules, and systems described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

Further embodiments herein may be formed by supplementing an embodiment with one or more element from any one or more other embodiment herein, and/or substituting one or more element from one embodiment with one or more element from one or more other embodiment herein.

It is understood, that the invention is defined by the appended claims.

**Claims**

1.  A system for automatically detecting a cardiac structure of interest, comprising:

    a mapping catheter having at least one sensing device configured to be positioned within the heart to receive electrophysiological data regarding a first cardiac structure, the at least one sensing device configured to provide a one-dimensional intracardiac electrogram or electrocardiogram signal;
    a reference catheter (1930) having at least one reference electrode configured to be brought into contact with an endocardial surface of the heart;
    a processor comprising a neural network that has been trained to identify automatically the cardiac structure of interest based on the one-dimensional signal and distance data, adapted to:

        receive the one-dimensional signal from the at least one sensing device;
        receive distance data regarding a distance between the at least one sensing device and the reference electrode;
        apply the neural network to the received one-dimensional signal to determine outputs;
        apply a weight and a bias to the distance;
        apply an activation function to the weighted and biased distance; and
        multiply the determined outputs by the output of the activation function to determine whether the first cardiac structure is the cardiac structure of interest based on the electrophysiological data and the distance data.

2.  The system of claim 1 wherein the plurality of sensing devices includes a plurality of different electrodes.

3.  The system of claim 1, wherein the neural network is a convolutional neural network or a recurrent neural network.

4.  A system for automatically detecting a cardiac structure of interest, comprising:

    a mapping catheter having at least one sensing device configured to be positioned within the heart to receive electrophysiological data regarding a first cardiac structure, the at least one sensing device configured to provide a one-dimensional intracardiac electrogram or electrocardiogram signal;
    a reference catheter having at least one reference electrode configured to be brought into contact with an endocardial surface of the heart;
    a processor comprising a first neural network configured to receive the one-dimensional signal from the at least one sensing device and apply the neural network to the received one-dimensional signal to determine outputs;
    the processor comprising a second neural network configured to receive the outputs and a plurality of scalar values regarding at least a distance between the at least one sensing device and the reference electrode, and to determine whether the first cardiac structure is the cardiac structure of interest based on the electrophysiological data and the distance data,
    wherein the first neural network and the second neural network are trained monolithically on the one-dimensional signals and the distance data to identify automatically the cardiac structure of interest.

5.  The system of claim 1 or claim 4, wherein the cardiac structure of interest comprises a His bundle.

6.  The system of claim 4 wherein the plurality of sensing devices includes a plurality of different electrodes.

7.  The system of claim 1 or claim 4 wherein the reference catheter has multiple electrodes.

**8.** The system of claim 4 wherein the first neural network is a convolutional neural network.

**9.** The system of claim 8 wherein the second neural network is a non-convolutional neural network having one or more hidden layers.

**Patentansprüche**

**1.** System zum automatischen Detektieren einer kardialen Struktur von Interesse, umfassend:

einen Kartierungskatheter aufweisend mindestens eine Erfassungsvorrichtung, die dazu konfiguriert ist, innerhalb des Herzens positioniert zu werden, um elektrophysiologische Daten bezüglich einer ersten Herzstruktur zu empfangen, wobei die mindestens eine Erfassungsvorrichtung dazu konfiguriert ist, ein eindimensionales intrakardiales Elektrogramm- oder Elektrokardiogrammsignal bereitzustellen;
einen Referenzkatheter (1930) aufweisend mindestens eine Referenzelektrode, die dazu konfiguriert ist, in Kontakt mit einer endokardialen Oberfläche des Herzens gebracht zu werden;
einen Prozessor, der ein neuronales Netz umfasst, das darauf trainiert wurde, die kardiale Struktur von Interesse basierend auf den eindimensionalen Signal- und Entfernungsdaten automatisch zu identifizieren, angepasst zum:

Empfangen des eindimensionalen Signals von der mindestens einen Erfassungsvorrichtung;
Empfangen von Abstandsdaten bezüglich eines Abstands zwischen der mindestens einen Erfassungsvorrichtung und der Referenzelektrode;
Anwenden des neuronalen Netzes auf das empfangene eindimensionale Signal, um Ausgaben zu bestimmen;
Anwenden einer Gewichtung und eines Bias auf den Abstand;
Anwenden einer Aktivierungsfunktion auf den gewichteten und mit einem Bias versehenen Abstand; und
Multiplizieren der bestimmten Ausgaben mit der Ausgabe der Aktivierungsfunktion , um zu bestimmen, ob die erste Herzstruktur die Herzstruktur von Interesse ist, basierend auf den elektrophysiologischen Daten und den Abstandsdaten.

**2.** System nach Anspruch 1, wobei die Vielzahl von Erfassungsvorrichtungen eine Vielzahl von verschiedenen Elektroden umfasst.

**3.** System nach Anspruch 1, wobei das neuronale Netz ein faltendes neuronales Netz oder ein rekurrentes neuronales Netz ist.

**4.** System zum automatischen Detektieren einer kardialen Struktur von Interesse, umfassend:

einen Kartierungskatheter aufweisend mindestens eine Erfassungsvorrichtung, die dazu konfiguriert ist, innerhalb des Herzens positioniert zu werden, um elektrophysiologische Daten bezüglich einer ersten Herzstruktur zu empfangen, wobei die mindestens eine Erfassungsvorrichtung dazu konfiguriert ist, ein eindimensionales intrakardiales Elektrogramm- oder Elektrokardiogrammsignal bereitzustellen;
einen Referenzkatheter mit mindestens einer Referenzelektrode, die dazu konfiguriert ist, in Kontakt mit einer endokardialen Oberfläche des Herzens gebracht zu werden;
einen Prozessor, der ein erstes neuronale Netz umfasst, das konfiguriert ist, das eindimensionale Signal von der mindestens einen Erfassungsvorrichtung zu empfangen und das neuronale Netz auf das empfangene eindimensionale Signal anzuwenden, um Ausgaben zu bestimmen;
wobei der Prozessor ein zweites neuronales Netz umfasst, das so konfiguriert ist, dass es die Ausgaben und eine Vielzahl von Skalarwerten bezüglich mindestens eines Abstands zwischen der mindestens einen Erfassungsvorrichtung und der Referenzelektrode empfängt und auf der Grundlage der elektrophysiologischen Daten und der Abstandsdaten bestimmt, ob die erste Herzstruktur die Herzstruktur von Interesse ist,
wobei das erste neuronale Netz und das zweite neuronale Netz monolithisch auf die eindimensionalen Signale und die Abstandsdaten trainiert werden, um die Herzstruktur von Interesse automatisch zu identifizieren.

**5.** System nach Anspruch 1 oder Anspruch 4, wobei die kardiale Struktur von Interesse ein His-Bündel umfasst.

**6.** System nach Anspruch 4, wobei die Vielzahl von Erfassungsvorrichtungen eine Vielzahl von verschiedenen Elektroden einschließt.

7. System nach Anspruch 1 oder Anspruch 4, wobei der Referenzkatheter mehrere Elektroden aufweist.

8. System nach Anspruch 4, wobei das erste neuronale Netz ein faltendes neuronales Netz ist.

9. System nach Anspruch 8, wobei das zweite neuronale Netz ein nichtfaltendes neuronales Netz mit einer oder mehreren verborgenen Schichten ist.

**Revendications**

1. Système de détection automatique d'une structure cardiaque d'intérêt, comprenant :

un cathéter de cartographie ayant au moins un dispositif de détection configuré pour être positionné dans le coeur afin de recevoir des données électrophysiologiques concernant une première structure cardiaque, l'au moins un dispositif de détection étant configuré pour fournir un signal d'électrogramme ou d'électrocardiogramme intracardiaque unidimensionnel ;
un cathéter de référence (1930) ayant au moins une électrode de référence configurée pour être mise en contact avec une surface endocardique du cœur ;
un processeur comprenant un réseau neuronal qui a été entraîné à identifier automatiquement la structure cardiaque d'intérêt sur la base du signal unidimensionnel et des données de distance, adapté pour :

recevoir le signal unidimensionnel de l'au moins un dispositif de détection ;
recevoir des données de distance concernant une distance entre l'au moins un dispositif de détection et l'électrode de référence ;
appliquer le réseau neuronal au signal unidimensionnel reçu afin de déterminer des sorties ;
appliquer une pondération et un biais à la distance ;
appliquer une fonction d'activation à la distance pondérée et biaisée ; et
multiplier les sorties déterminées par la sortie de la fonction d'activation afin de déterminer si la première structure cardiaque est la structure cardiaque d'intérêt ou non sur la base des données électrophysiologiques et des données de distance.

2. Système selon la revendication 1, dans lequel la pluralité de dispositifs de détection comporte une pluralité d'électrodes différentes.

3. Système selon la revendication 1, dans lequel le réseau neuronal est un réseau neuronal convolutif ou un réseau neuronal récurrent.

4. Système de détection automatique d'une structure cardiaque d'intérêt, comprenant :

un cathéter de cartographie ayant au moins un dispositif de détection configuré pour être positionné dans le cœur afin de recevoir des données électrophysiologiques concernant une première structure cardiaque, l'au moins un dispositif de détection étant configuré pour fournir un signal d'électrogramme ou d'électrocardiogramme intracardiaque unidimensionnel ;
un cathéter de référence comportant au moins une électrode de référence configurée pour être mise en contact avec une surface endocardique du cœur ;
un processeur comprenant un premier réseau neuronal configuré pour recevoir le signal unidimensionnel de l'au moins un dispositif de détection et appliquer le réseau neuronal au signal unidimensionnel reçu afin de déterminer des sorties ;
le processeur comprenant un second réseau neuronal configuré pour recevoir les sorties et une pluralité de valeurs scalaires concernant au moins une distance entre l'au moins un dispositif de détection et l'électrode de référence, et pour déterminer si la première structure cardiaque est la structure cardiaque d'intérêt ou non sur la base des données électrophysiologiques et des données de distance,
dans lequel le premier réseau neuronal et le second réseau neuronal sont entraînés de manière monolithique sur les signaux unidimensionnels et les données de distance afin d'identifier automatiquement la structure cardiaque d'intérêt.

**5.** Système selon la revendication 1 ou la revendication 4, dans lequel la structure cardiaque d'intérêt comprend un faisceau de His.

**6.** Système selon la revendication 4, dans lequel la pluralité de dispositifs de détection comporte une pluralité d'électrodes différentes.

**7.** Système selon la revendication 1 ou la revendication 4, dans lequel le cathéter de référence a de multiples électrodes.

**8.** Système selon la revendication 4, dans lequel le premier réseau neuronal est un réseau neuronal convolutif.

**9.** Système selon la revendication 8, dans lequel le second réseau neuronal est un réseau neuronal non convolutif ayant une ou plusieurs couches cachées.

FIG. 1

FIG. 2

FIG. 3

**400**

420

410

Training the Machine    450

Hardware

Data

430

Building a Model

440

Predicting Outcome

# FIG. 4

EP 3 918 999 B1

500

```
+------------------------------------+
|                                    |
|     Collect Data From Hardware     |----- 510
|                                    |
+------------------------------------+
                 |
                 v
+------------------------------------+
|                                    |
|    Train a Machine on the Hardware |----- 520
|                                    |
+------------------------------------+
                 |
                 v
+------------------------------------+
|                                    |
|            Build a Model           |----- 530
|                                    |
+------------------------------------+
                 |
                 v
+------------------------------------+
|                                    |
|   Predict Outcomes of the Hardware |----- 540
|                                    |
+------------------------------------+
```

# FIG. 5

610

| Weather | Play |
|---|---|
| Sunny | No |
| Overcast | Yes |
| Rainy | Yes |
| Sunny | Yes |
| Sunny | Yes |
| Overcast | Yes |
| Rainy | No |
| Rainy | No |
| Sunny | Yes |
| Rainy | Yes |
| Sunny | No |
| Overcast | Yes |
| Overcast | Yes |
| Rainy | No |

620

| Frequency Table | | |
|---|---|---|
| Weather | No | Yes |
| Overcast | | 4 |
| Rainy | 3 | 2 |
| Sunny | 2 | 3 |
| Grand Total | 5 | 9 |

630

| Likelihood Table | | | | |
|---|---|---|---|---|
| Weather | No | Yes | | |
| Overcast | | 4 | =4/14 | 0.29 |
| Rainy | 3 | 2 | =5/14 | 0.36 |
| Sunny | 2 | 3 | =5/14 | 0.36 |
| All | 5 | 9 | | |
| | =5/14 | =9/14 | | |
| | 0.36 | 0.64 | | |

FIG. 6

EP 3 918 999 B1

FIG. 7

EP 3 918 999 B1

FIG. 8

FIG. 9

FIG. 10

FIG. 11

FIG. 12

Bagging - Parallel                    Boosting - Sequential

FIG. 13

FIG. 14

**Neuron Block**

1500    1510

On-chip RAM

Data — Weights (w)
Address — Biases (b)
Write_En — Direction (d)
Register B — Sel_Addr

$x_0$  $x_1$  $x_2$  $x_3$    +1   Reg_A

Multiplexer    Sel_X

1520

1570    ×

1560

1530

En_Out — FSM Control Unit — Sleep
En_A — Reset
En_B
Sel_Opcode
Rst_ALU

Reg_B — Arithmetic Logic Unit

Reg_A —

Rst_Cnt
En_Cnt
Use_ALU_Reg    Count

1550

En_Out — $y_0$  $y_1$  $y_2$  $y_3$

Sel_Addr
Sel_X — Operand Selector

Sel_Y

1540    Clock

FIG. 15

40

**FIG. 16**

FIG. 17

1820

1800

1830  1840  1850

1810

FIG. 18A

1860

RECEIVE FIRST INPUT DATA
FROM FIRST CATHETER ⟶ 1865

RECEIVE ADDITIONAL INPUT DATA ⟶ 1875

APPLY MACHINE LEARNING ALGORITHM TO EACH
INPUT RECEIVED ⟶ 1885

GENERATE OUTPUT ⟶ 1890

TRAIN NEURAL NETWORK ⟶ 1895

FIG. 18B

FIG. 19A

FIG. 19B

FIG. 20

EP 3 918 999 B1

2100

2110    2120    2130

FIG. 21

FIG. 22

FIG. 23

FIG. 24

FIG. 25

**REFERENCES CITED IN THE DESCRIPTION**

**Patent documents cited in the description**

- US 2009253976 A1